# EUROPEAN PATENT APPLICATION

(11) **EP 1 449 532 A1**
(43) Date of publication of application: **25.08.2004**
(21) Application number: 04012293.9
(22) Date of filing: 18.08.2000
(51) Int. Cl.: A61K 31/519, A61K 31/505, A61K 31/522, A61K 31/4427, A61K 31/00, C07D 213/74

(54) **Compound [2-(4-chloro-2,6-dimethyl-phenoxy)- 3,6-dimethyl-pyridin-4-yl]- (1-ethyl-propyl)- amine and use as CRF antagonist**

(30) Priority: 27.08.1999 US 151183 P
(62) Divisional of application: 00307074.5
(71) Applicant: Pfizer Products Inc., Groton, Connecticut 06340 (US)
(72) Inventor: Chen, Yuhpyng Liang, Groton 06340 Connecticut (US)
(74) Representative: Eddowes, Simon

(57) **Abstract**

The compound [2-(4-chloro-2,6-dimethyl-phenoxy)-3,6-dimethyl-pyridin-4-yl]-(1-ethyl-propyl)-amine.

## Description

### Field of the Invention

This invention relates to the use of CRF antagonists in the treatment of certain conditions, and related compositions.

### Background Of The Invention

CRF antagonists are disclosed in U.S. Patents 4,605,642 (Issued August 12, 1986) and 5,063,245 (issued November 5, 1991). They are also disclosed in International patent publications WO 95/33750 (published December 14, 1995); WO 95/34563 (published December 21, 1995); WO 94/13676 (published June 23, 1994); WO 94/13677 (published June 23, 1994); WO 95/33727 (published December 14, 1995); WO 98/05661 (published February 12, 1998); WO 98/08847 (published March 5, 1998); and WO 98/08846 (published March 5, 1998) and European patent publications EP 778277 (published June 11, 1997) and EP 773023 (published May 14, 1997). CRF antagonists are also disclosed in the following patent publications: EP 576350; WO 95/10506 (published April 20, 1995); WO 96/35689 (published November 14, 1996); WO 96/39400 (published December 12, 1996); WO 97/00868 (published January 9, 1997); WO 97/14684 (published April 24, 1997); WO 97/29109 (published August 14, 1997); WO 97/29110 (published August 14, 1997); WO 97/35580 (published October 2, 1997); WO 97/35846 (published October 2, 1997), WO 97/44038 (published November 27, 1997); WO 98/03510 (published January 29, 1998); WO 98/08821 (published March 5, 1998); WO 98/11075 (published March 19, 1998), WO 98/15543 (published April 16, 1998); WO 98/21200 (published May 22, 1998); WO 98/27066 (published June 25, 1998); WO 98/29397 (published July 9, 1998); WO 98/29413 (published July 9, 1998); WO 98/42699 (published October 1, 1998); WO 98/35967 (published August 20, 1998); WO 98/45295 (published October 15, 1998); WO 98/47874 (published October 29, 1998); WO 98/47903 (published October 29, 1998); WO 99/01454 (published January 14, 1999); WO 99/01439 (published January 14, 1999); WO 99/10350 (published March 4, 1999), WO 99/12908 (published March 18, 1999); WO 99/00373 (published January 7, 1999); and WO 99/38868 (published August 5, 1999).

The importance of CRF antagonists is set out in the literature, e.g., P Black, Scientific American SCIENCE & MEDICINE,1995, p. 16-25; T. Lovenberg, et al., Current Pharmaceutical Design, 1995, 1: 305-316; and United States Patent 5,063,245. An outline of the activities possessed by CRF antagonists is found in M. J. Owens et al., 1991, Pharm. Rev., 43:425-473. CRF antagonists are described in the art as being effective in the treatment of stress-related illnesses, mood disorders such as depression, major depressive disorder, single episode depression, recurrent depression, child abuse induced depression, postpartum depression, dysthemia, bipolar disorders, and cyclothymia; chronic fatigue syndrome; eating disorders such as anorexia and bulimia nervosa; generalized anxiety disorder; panic disorder; phobias; obsessive-compulsive disorder; post-traumatic stress disorder; pain perception such as fibromyalgia; headache; gastrointestinal diseases; hemorrhagic stress; ulcers; stress-induced psychotic episodes; fever; diarrhea; post-operative ileus; colonic hypersensitivity; irritable bowel syndrome; Crohn's disease; spastic colon; inflammatory disorders such as rheumatoid arthritis and osteoarthritis; pain; asthma; psoriasis; allergies; osteoporosis; premature birth; hypertension, congestive heart failure; sleep disorders; neurodegenerative diseases such as Alzheimer's disease, senile dementia of the Alzheimer's type, multiinfarct dementia, Parkinson's disease, and Huntington's disease; head trauma; ischemic neuronal damage; excitotoxic neuronal damage; epilepsy; stroke; spinal cord trauma; psychosocial dwarfism; euthyroid sick syndrome; syndrome of inappropriate antidiarrhetic hormone; obesity; chemical dependencies and addictions; drug and alcohol withdrawal symptoms; infertility; cancer; muscular spasms; urinary incontinence; hypoglycemia and immune dysfunctions including stress induced immune dysfunctions, immune suppression and human immunodeficiency virus infections; and stress-induced infections in humans and animals.

### Summary Of The Invention

The present invention relates to a method of treating a condition comprising administering a corticotropin releasing factor (CRF) antagonist in an amount effective to treat the condition, the condition being selected from the group consisting of:
a) disorders that can be treated by altering circadian rhythm; and
b) depression, further wherein a second compound for treating depression is administered, the second compound for treating depression having an onset of action that is delayed with respect to that of the CRF antagonist.

In another aspect, the present invention relates to a method for treating or preventing a cardiovascular disease that involves administering a CRF antagonist in combination with a non-CRF antagonist compound for treating the disease. The invention also relates to treatment of migraine or non-migraine headache by administration of a CRF antagonist in combination with a non-CRF antagonist compound that treats such condition and to treatment of emesis using a CRF antagonist in combination with a non-CRF antagonist compound for treating emesis.

### Detailed Description of the Invention

All patents, patent publications, and literature references cited herein are hereby incorporated by reference.

In one aspect, the present invention provides for treatment of disorders that can be treated by altering circadian rhythm, e.g., abnormal circadian rhythm, by administration of a CRF antagonist. Abnormal circadian rhythm treated according to the invention can be associated with several types of disorders, including, without limitation, time zone change syndrome, seasonal affective disorder, irregular sleep-wake pattern, delayed sleep phase syndrome, advanced sleep phase syndrome, non-24 hour sleep wake disorder, light-induced clock· resetting, REM sleep disorder, hypersomnia, parasomnia, narcolepsy, nocturnal enuresis, restless legs syndrome, sleep apnea, dysthymia, and abnormal circadian rhythm associated with chronic administration and withdrawal of antidepressant agents.

If desired, a second compound, e.g., a non-CRF antagonist that is useful for treating sleep disorder, can be administered before, with, or after, administration of the CRF antagonist. Any such second compound useful for treating sleep disorder may be employed, including but not limited to tachykinin antagonists, melatoninergic agonists, such as melatonin, GABA brain receptor agonists, serotonin receptor (such as 5HT1 b, 5HT2c, 5HT7) antagonists, inverse agonists, agonists and other compounds. Specific compounds for treatment of sleep disorder include melatonin, carpipramine, and doxylamine. These and other compounds are described, for example, in United States Patents 5,908,932; 5,902,813; 5,883,094; 5,874,450; 5,849,781; 5,856,529; and 4,956,362.

It is intended that reference to particular compounds herein be interpreted to mean that the pharmaceutically acceptable salts and prodrugs of those compounds, may also be employed. Such reference is also intended to be interpreted that modified CRF antagonists may also be employed. For example, the invention encompasses use of a CRF antagonist linked to a non-CRF antagonist to form a prodrug which hydrolyzes upon administration to form active components.

The invention also encompasses treatment of depression with a CRF antagonist and with a second compound having delayed action for treating depression. According to this aspect of the invention, the CRF antagonist initiates treatment of the depression with a quick-acting effect, which treatment is supplemented by the delayed effect of the second compound.

Compounds for treating depression that have a delayed effect include, without limitation, compounds that are selective serotonin reuptake inhibitors (SSRIs), tricyclic antidepressants, norepinephrine reuptake inhibitors, noradrenaline reuptake inhibitors, lithium, α2-adrenoreceptor agonists, 5HT_{1A} inhibitors, and monoamine oxidase type A inhibitors. Examples include bupropion, sertraline, fluoxetine, trazodone, citalopram, fluvoxamine, paroxetine, venlafaxine, roboxetine, imipramine, amitriptyline, trimipramine, doxepin, desipramine, nortriptyline, protriptyline, amoxapine, clomipramine, maprotiline, brofaromine, milnacipran, and buspirone. It understood by those skilled in this art that compounds administered for treatment of depression may have other beneficial effects, such as ameliorating sleep disturbance or sexual dysfunction. Compounds having a delayed effect for treating depression also include the combination of an SSRI and 5HT₂ antagonist (such as risperidone) administered, for example, to patients who do not respond to SSRI therapy alone. Administration of these delayed-action compounds for treating depression is carried out using well-known dosages and formulations.

Any CRF antagonist can be used to practice the invention, including those that are described in U.S. Patents 4,605,642 and 5,063,245; International patent publications WO 95/33750; WO 95/34563; WO 94/13676; W094/13677; WO 95/33727; WO 98/05661; WO 98/08847; and WO 98/08846; and European patent publications EP 778277; and EP 773023. They also include those of the following patent publications: EP 576350; WO 95/10506; WO 96/35689; WO 96/39400; WO 97/00868; WO 97/14684; WO 97/29109; WO 97/29110; WO 97/35580; WO 97/35846; WO 97/44038; WO 98/03510; WO 98/08821; WO 98/11075; WO 98/15543; WO 98/21200; WO 98/27066; WO 98/29397; WO 98/29413; WO 98/42699; WO 98/35967; WO 98/45295; WO 98/47874, WO 98/47903, WO 99/01454, W099/01439, W099/10350; W099/12908; W099/00373, and WO 99/38868. As noted above, the texts of all of these publications are incorporated by reference herein in their entireties.

Following are listed particular examples of CRF antagonists that may be used in practicing the invention. It is understood that in the generic formulae employed below, the variables employed, e.g., "A", "B", "R₁", "R₂", etc. have the meanings attributed to them only in the particular Roman numeral section in which they are found. Thus, the meaning attributed, for example, to "R¹" is different for the structures in section and the structures of the other sections.
**I.** For example, the CRF antagonist may be of the following formula, described in WO 94/13677: 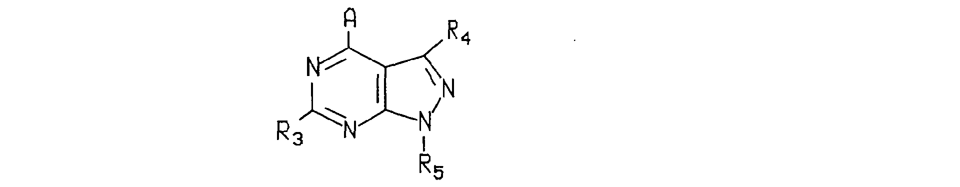 and the pharmaceutically acceptable acid addition salts thereof, wherein
   A is NR₁R₂, CR₁R₂R₁₁, or C(=CR₁R₁₂)R₂, NHCR₁R₂R₁₁, OCR₁R₂R₁₁, SCR₁R₂R₁₁, NHNR₁R₂, CR₂R₁₁NHR₁, CR₂R₁₁OR₁, CR₂R₁₁SR₁ or C(O)R₂;
   R₁ is hydrogen, or C₁-C₆ alkyl which may be substituted by one or two substituents R₆ independently selected from the group consisting of hydroxy, fluoro, chloro, bromo, iodo, C₁-C₆ alkoxy, O-C(O)-(C₁-C₆ alkyl), O-C(O)-N(C₁-C₄ alkyl)(C₁-C₂ alkyl); amino, NH(C₁-C₄ alkyl), S(C₁-C₆ alkyl), OC(O)NH(C₁-C₄ alkyl), N(C₁-C₂ alkyl)C(O)(C₁-C₄ alkyl), NHC(O)(C₁-C₄ alkyl), COOH, CO(C₁-C₄ alkyl), C(O)NH(C₁-C₄ alkyl), C(O)N(C₁-C₄ alkyl)(C₁-C₂ alkyl), SH, CN, NO₂, SO(C₁-C₄ alkyl); SO₂(C₁-C₄ alkyl), SO₂NH(C₁-C₄ alkyl), SO₂N(C₁-C₄ alkyl)(C₁-C₂ alkyl), and said C₁-C₆ alkyl may have one or two double or triple bonds;
   R² is C₁-C₁₂ alkyl, aryl or (C₁-C₁₀ alkylene)aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl; 3- to 8-membered cycloalkyl or (C₁-C₆ alkylene) cycloalkyl, wherein said cycloalkyl may have one or two of O, S or N-Z, wherein Z is hydrogen, substituted , independently, for one or two carbons of said cycloalkyl, C₁-C₄ alkyl, benzyl or C₁-C₄ alkanoyl, wherein R² may be substituted independently by from one to three of chloro, fluoro, or C₁-C₄ alkyl, or one of hydroxy, bromo, iodo, C₁-C₆ alkoxy, OC(O)(C₁-C₆ alkyl), O-C-N(C₁-C₄ alkyl)(C₁-C₂ alkyl), S(C₁-C₆ alkyl), NH₂, NH(C₁-C₂ alkyl), N(C₁-C₄ alkyl) C(O)(C₁-C₄ alkyl), NHC(O)(C₁-C₄ alkyl), COOH, C(O)O(C₁-C₄ alkyl), C(O)NH(C₁-C₄ alkyl), C(O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl), SO₂N(C₁-C₄ alkyl)(C₁-C₂ alkyl), and wherein said C₁-C₁₂ alkyl or C₁-C₁₀ alkylene may have one to three double or triple bonds; or
   NR₁R₂ or CR₁R₂R₁₁ may form a 4- to 8-membered ring optionally having one or two double bonds or one or two of O, S or N-Z wherein Z is hydrogen, C₁-C₄ alkyl, benzyl, or C₁-C₄ alkanoyl;
   R₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl and C₁-C₆ alkyl may have one or two double or triple bonds and may be substituted by from 1 to 3 R₇ substituents independently selected from the group consisting of hydroxy, amino, C₁-C₃ alkoxy, dimethylamino, diethylamino, methylamino. ethylamino, NHC(O)CH₃, fluoro, chloro or C₁-C₃ thioalkyl;
   R₄ is hydrogen, C₁-C₆ alkyl, fluoro, chloro, bromo, iodo, C₁-C₆ alkoxy, amino, NH(C₁-C₆ alkyl), N(C₁-C₆ alkyl) (C₁-C₂ alkyl), SOₙ(C₁-C₆ alkyl), wherein n is 0, 1 or 2, cyano, hydroxy, carboxy, or amido, wherein said C₁-C₆ alkyls may be substituted by one to three of hydroxy, amino, carboxy, amido, NHC(O)(C₁-C₄ alkyl), NH(C₁-C₄ alkyl), N(C₁-C₄ alkyl)(C₁-C₂ alkyl), C(O)O(C₁-C₄ alkyl), C₁-C₃ alkoxy, C₁-C₃ thioalkyl, fluoro, bromo, chloro, iodo, cyano or nitro;
   R₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl benzoxazolyl, oxazolyl, pyrrolidinyl, thiazolidinyl, piperazinyl, piperidinyl, or tetrazolyl, wherein each one of the above groups may be substituted independently by from one to three of fluoro, chloro, bromo, formyl, C₁-C₆ alkyl, C₁-C₆ alkoxy or trifluoromethyl, or one of hydroxy, iodo, cyano, nitro, amino, cyclopropyl, NH(C₁-C₄ alkyl), N(C₁-C₄ alkyl)(C₁-C₂ alkyl), COO(C₁-C₄ alkyl), CO(C₁-C₄ alkyl), SO_{z}NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl and C₁-C₆ alkyl may have one double or triple bond and may be substituted by one or two of fluoro, chloro, hydroxy, amino, methylamino, dimethylamino or acetyl; with the proviso that R₅ is not unsubstituted phenyl;
   R₁₁ is hydrogen, hydroxy, fluoro, chloro, COO(C₁-C₂ alkyl), cyano, or CO(C₁-C₂ alkyl); and
   R₁₂ is hydrogen or C₁-C₄ alkyl;
      (a) A is not straight chain C₁-C₁₂ alkyl;
      (b) when R₃ is hydrogen, A is benzyl or phenethyl, and R₄ is fluoro, chloro, bromo or iodo, then R₅ is not 5'-deoxy-ribofuranosyl or 5'-amino-5'-deoxy-ribofuranosyl; and
      (c) when R⁵ is phenyl, said phenyl is substituted by two or three substituents.
**II**. The invention also relates to use of a CRF antagonist of the following formula, described in WO 94/13676: 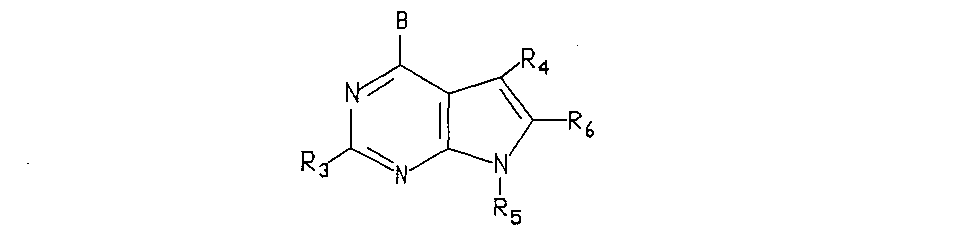 and the acid addition salts thereof, wherein
   B is XA wherein X is (CH₂)ₙ in which n is 0, 1 or 2, NH, 0, S, N(C₁-C₄ alkyl);
   A is NR₁R₂, CR₁R₂R₁₁, or C(=CR₂R₁₂)R₁;
   R₁ is hydrogen, or C₁-C₆ alkyl which may be substituted by one or two substituents R₇ independently selected from the group consisting of hydroxy, fluoro, chloro, bromo, iodo, C₁-C₈ alkoxy, O-C(=O)-(C₁-C₆ alkyl), O-C(=O)NH(C₁-C₄ alkyl), O-C(=O)-N(C₁-C₄ alkyl)(C₁-C₂ alkyl), amino, NH(C₁-C₄ alkyl), N(C₁-C₂ alkyl)(C₁-C₄ alkyl), S(C₁-C₆ alkyl), N(C₁-C₄alkyl)C(=O)(C₁-C₄ alkyl), NH(C₁-C₄ alkyl), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl), SO₂N(C₁-C₄ alkyl)(C₁-C₂ alkyl), and said C₁-C₆ alkyl may contain one or two double or triple bonds;
   R₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl, or benzoxazolyl; 3-to 8-membered cycloalkyl or (C₁-C₆ alkylene) cycloalkyl, wherein said cycloalkyl may contain one or two of O, S or N-Z wherein Z is hydrogen, C₁-C₄ alkyl, benzyl or C₁-C₄ alkanoyl, wherein R₂ may be substituted independently by from one to three of chloro, fluoro, or C₁-C₄ alkyl, or one of hydroxy, bromo, iodo, C₁-C₆ alkoxy, O-C(=O)(C₁-C₆ alkyl), O-C-N(C₁-C₄ alkyl)(C₁-C₂ alkyl), S(C₁-C₆ alkyl), NH₂, NH(C₁-C₂ alkyl), N(C₁-C₂ alkyl) (C₁-C₄ alkyl), N(C₁-C₄)- C(=O)(C₁-C₄ alkyl), NHC(=O)(C₁-C₄), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl), SO₂N(C₁-C₄ alkyl)(C₁-C₂ alkyl), and wherein said C₁-C₁₂ alkyl or C₁-C₁₀ alkyl may contain one to three double or triple bonds; or
   when A is NR₁R₂ or CR₁R₂R₁₁, then R₁ and R₂ taken together with the atom to which they are attached may form a saturated 4- to 8-membered optionally containing one or two double bonds or one or two of O, S or N-Z wherein Z is hydrogen, C₁-C₄ alkyl, or C₁-C₄ alkanoyl;
   R₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl and C₁-C₆ alkyl may contain from one or two double or triple bonds and may be substituted by from 1 to 3 substituents R₈ independently selected from the group consisting of hydroxy, amino, C₁-C₃ alkoxy, dimethylamino, diethylamino, methylamino, ethylamino, NHCH₃, fluoro, chloro or C₁-C₃ thioalkyl;
   R₄ and R₆ are each independently hydrogen, C₁-C₆ alkyl, fluoro, chloro, bromo, iodo, C₁-C₆ alkoxy, amino, NH(C₁-C₆ alkyl), N(C₁-C₆ alkyl)(C₁-C₂ alkyl), SOₙ(C₁-C₆ alkyl), wherein n is 0, 1 or 2, cyano, hydroxy, carboxy, or amido, wherein said C₁-C₆ alkyls may be substituted by one to three of hydroxy, amino, carboxy, amido, NHC(=O)(C₁-C₄ alkyl), NH(C₁-C₄ alkyl), N(C₁-C₄ alkyl)(C₁-C₂ alkyl), C(=O)O(C₁-C₄ alkyl), C₁-C₃ alkoxy, C₁-C₃ thioalkyl, fluoro, bromo, chloro, iodo, cyano or nitro;
   R₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, piperidinyl, piperazinyl, tetrazolyl, or 3- to 8-membered cycloalkyl or 9- to 12-membered bicycloalkyl, optionally containing one to three of O, S or N-Z wherein Z is hydrogen, C₁-C₄ alkyl, C₁-C₄ alkanoyl, phenyl or phenylmethyl, wherein each one of the above groups may be substituted independently by from one to four of fluoro, chloro, C₁-C₆ alkyl, C₁-C₆ alkoxy or trifluoromethyl, or one of bromo, iodo, cyano, nitro, amino, NH(C₁-C₄ alkyl), N(C₁-C₄)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl and C₁-C₆ alkyl may be substituted by one or two of fluoro, chloro, hydroxy, amino, methylamino, dimethylamino or acetyl; with the proviso that R₅ is not unsubstituted phenyl;
   R₁₁ is hydrogen, hydroxy, fluoro, chloro, COO(C₁-C₂ alkyl), cyano, or CO(C₁-C₂ alkyl); and
   R₁₂ is hydrogen or C₁-C₄ alkyl; with the proviso that (1) when R₅ is 4-bromophenyl, R₃ is hydrogen, and R₄ and R₆ are methyl, then B is not methylamino or ethyl, and (2) when R₅ is 4-bromophenyl, and R₃, R₄ and R₆ are methyl, then B is not 2-hydroxyethylamino.
**III.** It is also possible to employ a CRF antagonist that has a structure selected from the group shown below, and pharmaceutically acceptable salts and esters thereof, as described in WO 95/33750: 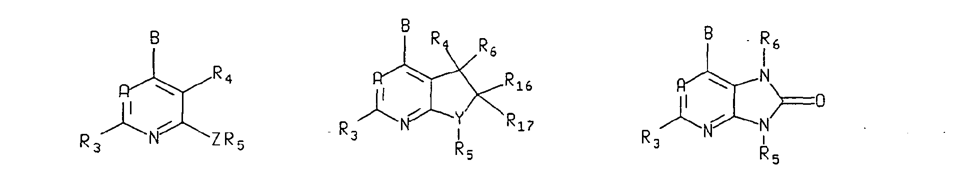 wherein
   A is CR₇ or N;
   B is NR₁R₂, CR₁R₂R₁₁, C(=CR₂R₁₂)R₁, NHCHR₁R₂, OCHR₁R₂, SCHR₁R₂, CHR₂OR₁₂, CHR₂SR₁₂, C(S)R₂ or C(O)R₂;
   Y is CH or N;
   Z is NH, O, S, N (C₁-C₂ alkyl), or CR₁₃R₁₄, wherein R₁₃ and R₁₄ are each independently hydrogen, trifluoromethyl, or C₁-C₄ alkyl, or one of R₁₃ and R₁₄ may be cyano, chloro, bromo, iodo, fluoro, hydroxy, O(C₁-C₂ alkyl), amino, NH(C₁-C₂ alkyl), or CR₁₃R₁₄ may be C=O or cyclopropyl;
   R₁ is C₁-C₆ alkyl which may be substituted by one or two substituents R₈ independently selected from the group consisting of hydroxy, fluoro, chloro, bromo, iodo, C₁-C₄ alkoxy, O-CO-(C₁-C₄ alkyl), O-CO-NH(C₁-C₄ alkyl), O-CO-N(C₁-C₄ a)ky))(C₁-C₂ alkyl), NH(C₁-C₄ alkyl), N(C₁-C₂ alkyl)(C₁-C₄ alkyl), S(C₁-C₄ alkyl), N(C₁-C₄alkyl)CO(C₁-C₄ alkyl), NHCO(C₁-C₄ alkyl), COO(C₁-C₄ alkyl), CONH(C₁-C₄ alkyl), CON(C₁-C₄ alkyl)(C₁-C₂ alkyl), S(C₁-C₄ alkyl), CN, NO₂, SO(C₁-C₄ alkyl), SO₂(C₁-C₄ alkyl), and said C₁-C₆ alkyl or C₁-C₄ alkyl may contain one double or triple bond;
   R₂ is C₁-C₁₂ alkyl, aryl or (C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, or benzoxazolyl; 3- to 8-membered cycloalkyl or (C₁-C₆ alkylene)cycloalkyl, wherein said cycloalkyl may contain one or two of O, S or N-R₉ wherein R₉ is hydrogen, or C₁-C₄ alkyl, wherein the above defined R₂ may be substituted independently by from one to three of chloro, fluoro, or C₁-C₄ alkyl, or one of bromo, iodo, C₁-C₆ alkoxy, O-CO-(C₁-C₆ alkyl), O-CO-N(C₁-C₄ alkyl)(C₁-C₂ alkyl), S(C₁-C₆ alkyl), CN, NO₂, SO(C₁-C₄ alkyl), or SO₂(C₁-C₄ alkyl), and wherein said C₁-C₁₂ alkyl or C₁-C₄ alkylene may contain one double or triple bond; or
   NR₁R₂ or CR₁R₂R₁₁ may form a saturated 5- to 8-membered carbocyclic ring which may contain one or two double bonds or one or two of O or S;
   R₃ is methyl, ethyl, fluoro, chloro, bromo, iodo, cyano, methoxy, OCF₃, methylthio, methylsulfonyl, CH₂OH or CH₂OCH₃;
   R₄ is hydrogen, C₁-C₄ alkyl, fluoro, chloro, bromo, iodo, C₁-C₄ alkoxy, amino, nitro, NH(C₁-C₄ alkyl), N(C₁-C₄ alkyl)(C₁-C₂ alkyl), SOₙ(C₁-C₄ alkyl), wherein n is 0, 1 or 2, cyano, hydroxy, CO(C₁-C₄ alkyl), CHO, or COO(C₁-C₄ alkyl), wherein said C₁-C₄ alkyl may contain one or two double or triple bonds and may be substituted by one or two of hydroxy, amino, carboxy, NHCOCH₃, NH(C₁-C₂ alkyl), N(C₁-C₂ alkyl)₂, COO(C₁-C₄ alkyl), CO(C₁-C₄ alkyl), C₁-C₃ alkoxy, C₁-C₃ thioalkyl, fluoro, chloro, cyano or nitro;
   R₅ is phenyl, naphthyl, thienyl, benzothienyl, pyridyl, quinolyl, pyrazinyl, pyrimidyl, furanyl, benzofuranyl, benzothiazolyl, or indolyl, wherein each one of the above groups R₅ is substituted independently by from one to three of fluoro, chloro, C₁-C₆ alkyl, or C₁-C₆ alkoxy, or one of hydroxy, iodo, bromo, formyl, cyano, nitro, trifluoromethyl, amino, NH(C₁-C₄ alkyl), N(C₁-C₆)(C₁-C₂ alkyl), COOH, COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), or SO₂(C₁-C₆ alkyl), wherein said C₁-C₄ alkyl and C₁-C₆ alkyl may be substituted by one or two of fluoro, hydroxy, amino, methylamino, dimethylamino or acetyl;
   R₆ is hydrogen, or C₁-C₆ alkyl, wherein said C₁-C₆ alkyl may be substituted by one hydroxy, methoxy, ethoxy or fluoro;
   R₇ is hydrogen, C₁-C₄ alkyl, fluoro, chloro, bromo, iodo, cyano, hydroxy, O(C₁-C₄ alkyl), C(O)(C₁-C₄ alkyl), or C(O)O(C₁-C₄ alkyl), wherein the C₁-C₄ alkyl groups may be substituted with one hydroxy, chloro or bromo, or one to three fluoro;
   R₁₁ is hydrogen, hydroxy, fluoro, or methoxy;
   R₁₂ is hydrogen or C₁-C₄ alkyl; and
   R₁₆ and R₁₇ are each independently hydrogen, hydroxy, methyl, ethyl, methoxy, or ethoxy, except that they are not both methoxy or ethoxy, and CR₄R₆ and CR₁₆R₁₇ each independently may be C=O.
**IV.** It also possible to employ a CRF antagonist of the following formula, disclosed in WO 95/34563: 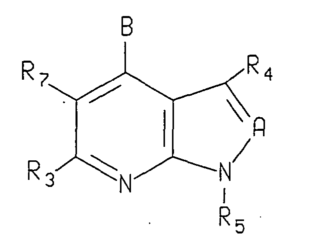 and the pharmaceutically acceptable acid addition salts thereof, wherein
   A is N or -CR₆;
   B is -NR₁R₂, -CR₁R₂R₁₁, -C(=CR₂R₁₂)R₁, -NHCHR₁R₂, -OCHR₁R₂, -SCHR₁R₂, -CHR₂OR₁₂, -CHR₂SR₁₂, -C(S)R₁ or-C(O)R₁;
   R₁ is C₁-C₆ alkyl which may optionally be substituted with one or two substituents independently selected from the group consisting of hydroxy, fluoro, chloro, bromo, iodo, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl), -SO₂(C₁-C₄ alkyl), and wherein any of the foregoing C₁-C₄ alkyl and C₁-C₆ alkyl groups may optionally contain one carbon-carbon double or triple bond;
   R₂ is C₁-C₁₂ alkyl, aryl, -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, oxazolyl, or benzoxazolyl; or 3- to 8-membered cycloalkyl or -(C₁-C₆ alkylene)cycloalkyl, wherein one or two of the ring carbons of said cycloalkyl having at least 4 ring members and the cycloalkyl moiety of said -(C₁-C₆ alkylene)cycloalkyl having at least 4 ring members may optionally be replaced by an oxygen or sulfur atom or by N-Z wherein Z is hydrogen; or C₁-C₄ alkyl, and wherein each of said groups R₂ may optionally be substituted with from one to three substituents independently selected from chloro, fluoro, and C₁-C₄ alkyl, or by one substituent selected from bromo, iodo, C₁-C₆ alkoxy, -O-CO-(C₁-C₆ alkyl), -S(C₁-C₆ alkyl), -COO(C₁-C₄ alkyl), CN, NO₂, -SO(C₁-C₄ alkyl), and -SO₂(C₁-C₄ alkyl), and wherein said C₁-C₁₂ alkyl and the C₁-C₄ alkylene moiety of said -(C₁-C₄ alkylene)aryl may optionally contain one carbon-carbon double or triple bond;
   or -NR₁R₂ may form a saturated 5- to 8-membered heterocyclic ring, or -CHR₁R₂ may form a saturated 5- to 8-membered carbocyclic ring, wherein each of these rings may optionally contain one or two carbon-carbon double bonds and wherein one or two of the carbon atoms of each of these rings may optionally be replaced with a sulfur or oxygen atom;
   R₃ is C₁-C₄ alkyl, fluoro, chloro, bromo, iodo, -CH₂OH, -CH₂OCH₃, -O(C₁-C₃ alkyl), -S(C₁-C₃ alkyl), or -SO₂(C₁-C₃ alkyl), wherein said C₁-C₃ alkyl may optionally contain one carbon-carbon double or triple bond;
   R₄ is hydrogen, C₁-C₆ alkyl, fluoro, chloro, bromo, iodo, C₁-C₄ alkoxy, amino, -NHCH₃, -N(CH₃)₂, -CH₂OH, -CH₂OCH₃, or -SOₙ(C₁-C₄ alkyl), wherein n is 0, 1 or 2, cyano, hydroxy, -CO(C₁-C₄ alkyl), -CHO, or -COO(C₁-C₄ alkyl) wherein the C₁-C₄ alkyl moieties in the foregoing R₄ groups may optionally contain one carbon-carbon double or triple bond;
   R₅ is phenyl, naphthyl, thienyl, benzothienyl, pyridyl, pyrimidyl, benzofuranyl, pyrazinyl or benzothiazolyl, wherein each one of said groups R₅ may optionally be substituted with from one to three substituents independently selected from fluoro, chloro, C₁-C₆ alkyl and C₁-C₆ alkoxy, or by one substituent selected from iodo, hydroxy, bromo, formyl, cyano, nitro, amino, trifluoromethyl, -NH(C₁-C₄ alkyl), -N(C₁-C₆)(C₁-C₂ alkyl), -COO(C₁-C₄ alkyl), -CO(C₁-C₄ alkyl), -COOH, -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), wherein each of said C₁-C₄ alkyl and C₁-C₆ alkyl moieties in the foregoing R⁵ groups may optionally be substituted with one to three fluorine atoms;
   R₆ is hydrogen, C₁-C₄ alkyl, fluoro, chloro, bromo, iodo, -CH₂OH, -CH₂OCH₃, or C₁-C₄ alkoxy;
   R₇ is hydrogen, C₁-C₄ alkyl, fluoro, chloro, bromo, iodo, -O(C₁-C₄ alkyl), cyano, -CH₂OH, -CH₂O(C₁-C₂ alkyl), -CO(C₁-C₂ alkyl), or -COO(C₁-C₂ alkyl);
   R₁₁ is hydrogen, hydroxy, fluoro, or methoxy; and
   R₁₂ is hydrogen or C₁-C₄ alkyl;
   with the proviso that when A is N, then: (a) B is not unsubstituted alkyl; (b) R₅ is not unsubstituted phenyl or monosubstituted phenyl; and (c) R₃ is not unsubstituted alkyl;
   or a pharmaceutically acceptable salt of such compound.
**V.** In another embodiment, the CRF antagonist is of the following formula, disclosed in EP 778277: 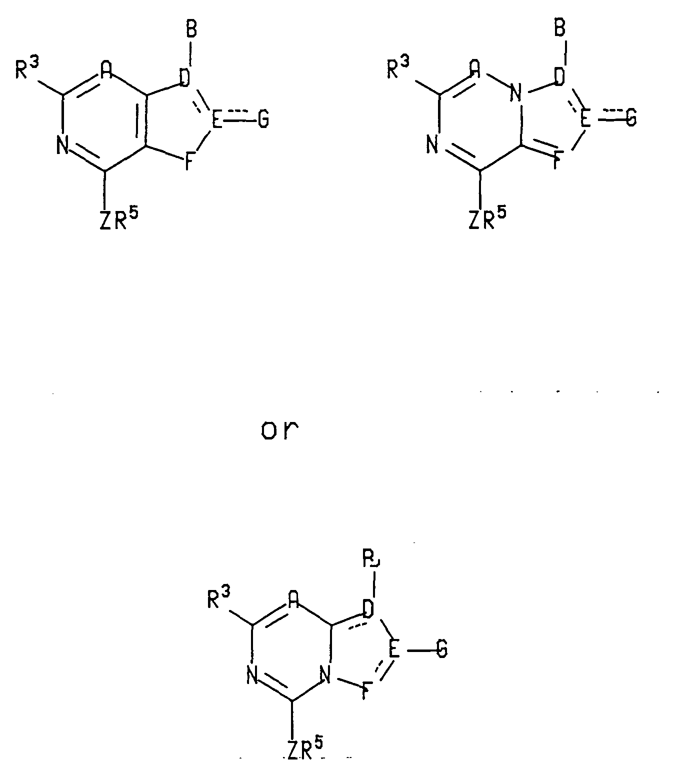 or a pharmaceutically acceptable salt thereof, wherein
   the dashed lines represent optional double bonds;
   A is nitrogen or CR⁷;
   B is -NR¹R², -CR¹R²R¹⁰, -C(=CR²R¹¹)R¹, -NHCR¹R²R¹⁰, -OCR¹R²R¹⁰, -SCR¹R²R¹⁰,-CR²R¹⁰NHR¹, -CR²R¹⁰OR¹, -CR²R¹⁰SR¹ or -COR ²;
   D is nitrogen and is single bonded to all atoms to which it is attached, or D is carbon and is either double bonded to E in formulas I and II or double bonded to the adjacent carbon atom common to both fused rings in formula III, or D is CH and is single bonded to E in formulas I and II;
   E is nitrogen, CH or carbon;
   F is oxygen, sulfur, CHR⁴ or NR⁴ when it is single bonded to E and F is nitrogen or CR⁴ when it is double bonded to E;
   G, when single bonded to E, is hydrogen, C₁-C₄ alkyl, -S(C₁-C₄ alkyl), -O(C₁-C₄ alkyl), NH_{2,} -NH(C₁-C₄ alkyl) or -N(C₁-C₂ alkyl)(C₁-C₄ alkyl), wherein each of the C₁-C₄ alkyl groups of G may optionally be substituted with one hydroxy, -O(C₁-C₂ alkyl) or fluoro group; G, when double bonded to E, is oxygen, sulfur or NH; and G, when E is nitrogen and double bonded to D or F, is absent;
   R¹ is hydrogen, C₁-C₆ alkyl optionally substituted with one or two substituents R⁸ independently selected from hydroxy, fluoro, chloro, bromo, iodo, C₁-C₄ alkoxy, CF₃, -C(=O)0-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
   R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl) may optionally and independently be replaced by an oxygen or sulfur atom or by NZ² wherein Z² is selected from hydrogen, C₁-C₄ alkyl, benzyl and C₁-C₄ alkanoyl, and wherein each of the foregoing R² groups may optionally be substituted with from one to three substituents independently selected from chloro, fluoro, hydroxy and C₁-C₄ alkyl, or with one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR¹R² or CR¹R²R¹⁰ may form a saturated 3 to 8 membered carbocyclic ring which may optionally contain from one to three double bonds and wherein one or two of the ring carbon atoms of such 5 to 8 membered rings may optionally and independently be replaced by an oxygen or sulfur atom or by NZ³ wherein Z³ is hydrogen, C₁-C₄ alkyl, benzyl or C₁-C₄ alkanoyl;
   R³ is hydrogen, C₁-C₄ alkyl, -O(C₁-C₄ alkyl), chloro, fluoro, bromo, iodo, -CN, -S(C₁-C₄ alkyl) or -SO₂(C₁-C₄ alkyl) wherein each of the (C₁-C₄ alkyl) moieties in the foregoing R³ groups may optionally be substituted with one substituent R⁹ selected from hydroxy, fluoro and (C₁-C₂ alkoxy);
   each R⁴ is, independently, hydrogen, (C₁-C₆ alkyl), fluoro, chloro, bromo, iodo, hydroxy, cyano, amino, nitro, -O(C₁-C₄ alkyl), -N(C₁-C₄ alkyl)(C₁-C₂ alkyl), -S(C₁-C₄ alkyl), -SO(C₁-C₄ alkyl), -SO₂(C₁-C₄)alkyl, -CO(C₁-C₄ alkyl), -C(=O)H or -C(=O)O(C₁-C₄alkyl), wherein each of the (C₁-C₆ alkyl) and (C₁-C₄ alkyl) moieties in the foregoing R⁴ groups may optionally contain one or two double or triple bonds and may optionally be substituted with one or two substituents independently selected from hydroxy, amino, C₁-C₃ alkoxy, dimethylamino, methylamino, ethylamino, -NHC(=O)CH₃, fluoro, chloro, C₁-C₃ thioalkyl, -CN, -COOH, -C(=O)O(C₁-C₄ alkyl), -C(=O)(C₁-C₄ alkyl) and -NO₂;
   R⁵ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or C₃-C₈ cycloalkyl wherein one or two of the carbon atoms of said cycloalkyl rings that contain at least 5 ring members may optionally and independently be replaced by an oxygen or sulfur atom or by NZ⁴ wherein Z⁴ is hydrogen, C₁-C₄ alkyl or benzyl; and wherein each of the foregoing R⁵ groups is substituted with from one to four substituents R¹² wherein one to three of said substituents may be selected, independently, from chloro, C₁-C₆ alkyl and -O(C₁-C₆ alkyl) and one of said substituents may be selected from bromo, iodo, formyl, -CN, -CF₃, -NO₂, -NH₂, -NH(C₁-C₄ alkyl), -N(C₁-C₂ alkyl)(C₁-C₆, alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
   R⁷ is hydrogen, C₁-C₄ alkyl, halo, cyano, hydroxy, -O(C₁-C₄ alkyl) -C(=O)(C₁-C₄ alkyl), -C(=O)O(C₁-C₄alkyl), -OCF₃, -CF₃, -CH₂OH, -CH₂O(C₁-C₄ alkyl);
   R¹⁰ is hydrogen, hydroxy, methoxy or fluoro;
   R¹¹ is hydrogen or C₁-C₄ alkyl; and
   Z is NH, oxygen, sulfur, -N(C₁-C₄ alkyl), -NC(=O)(C₁-C₂ alkyl), NC(=O)O(C₁-C₂alkyl) or CR¹³R¹⁴ wherein R¹³ and R¹⁴ are independently selected from hydrogen, trifluoromethyl and methyl with the exception that one of R¹³ and R¹⁴can be cyano;
   with the proviso that: (a) in the five membered rings of structures I, II and III, there can not be two double bonds adjacent to each other; and (b) when R⁴ is attached to nitrogen, it is
   not halo, cyano or nitro;
   or a pharmaceutically acceptable salt of such compound.
**VI.** The CRF antagonist can also be of the following formula, disclosed in WO 98/05661: 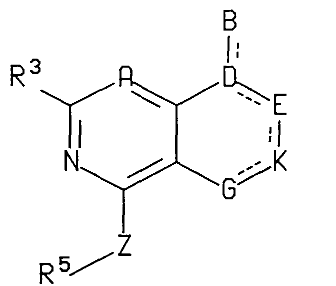 wherein the dashed lines represent optional double bonds;
   A is nitrogen or CR⁷;
   B is -NR¹R², -CR¹R²R¹⁰, -C(=CR ²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ or -COR², and is single bonded to D; or B is -CR¹R², and is double bonded to D and D is carbon;
   D is nitrogen or CR⁴ and is single bonded to all atoms to which it is attached, or D is carbon and is double bonded to E or double bonded to B;
   E is oxygen, nitrogen, sulfur, C=O, C=S, CR⁶R¹², NR⁶ or CR⁶; or E is a two atom spacer, wherein one of the atoms is oxygen, nitrogen, sulfur, C=O, C=S, CR⁶R¹², NR⁶ or CR⁶, and the other is CR⁶R¹² or CR⁹;
   K and G are each, independently, C=O, C=S, sulfur, oxygen, CHR⁸ or NR⁸ when single bonded to both adjacent ring atoms, or nitrogen or CR⁸ when it is double bonded to an adjacent ring atom;
   the 6- or 7-membered ring that contains D, E, K and G may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
   R¹ is C₁-C₆ alkyl optionally substituted with from one or two substituents independently selected from hydroxy, fluoro, chloro, bromo, iodo, C₁-C₄ alkoxy, CF₃, -C(=O)(C₁-C₄alkyl), -C(=O)-O-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
   R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl may optionally and independently be replaced by an oxygen or sulfur and wherein each of the foregoing R² groups may optionally be substituted with from one to three substituents independently selected from chloro, fluoro, hydroxy and C₁-C₄ alkyl, or with one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR¹R² or CR¹R²R¹⁰ may form a ring selected from saturated 3 to 8 membered rings, the 5 to 8 membered rings of which may optionally contain one or two double bonds, and wherein one or two of the ring carbon atoms of such 5 to 8 membered rings may optionally and independently be replaced by an oxygen or sulfur atom or by NZ³ wherein Z³ is hydrogen or C₁-C₄ alkyl;
   R³ is hydrogen, C₁-C₄ alkyl, -O(C₁-C₄ alkyl), chloro, fluoro, bromo, iodo, -S(C₁-C₄ alkyl) or -SO₂(C₁-C₄ alkyl);
   R⁴ is hydrogen, C₁-C₂ alkyl, hydroxy or fluoro;
   each R⁶, R⁸ and R⁹ that is attached to a carbon atom is selected, independently, from hydrogen, C₁-C₂ alkyl, fluoro, chloro, bromo, iodo, hydroxy, hydroxymethyl, formyl, trifluoromethyl, cyano, amino, nitro, -O(C₁-C₂ alkyl), -N(C₁-C₂ alkyl)(C₁-C₂ alkyl), -S(C₁-C₂ alkyl), -CO(C₁-C₂ alkyl), -C(=O)H or -C(=O)O(C₁-C₂ alkyl), wherein each of the C₁-C₂ alkyl moieties in the foregoing R⁶, R⁸, and R⁹ groups may optionally contain one double or triple bond; and each R⁶, R⁸, and R⁹ that is attached to a nitrogen atom is selected, independently, from hydrogen and C₁-C₄ alkyl;
   R⁵ is substituted phenyl, naphthyl, pyridyl or pyrimidyl, wherein each of the foregoing R⁵ groups is substituted with from two to four substituents R¹⁵, wherein from one to three of said substituents may be selected, independently, from chloro, C₁-C₆ alkyl, -O(C₁-C₆ alkyl) and -(C₁-C₆alkylene)O(C₁-C₆alkyl), and wherein one of said substituents may be selected, independently, from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
   R⁷ is hydrogen, methyl, halo (e.g., chloro, fluoro, iodo or bromo), hydroxy, methoxy, -C(=O)(C₁-C₂ alkyl), -C(=O)O(C₁-C₂ alkyl), trifluoromethoxy, hydroxymethyl, trifluoromethyl or formyl;
   R¹⁰ is hydrogen, hydroxy, methoxy or fluoro;
   R¹¹ is hydrogen or C₁-C₄ alkyl;
   R¹² is, hydrogen or methyl; and
   Z is NH, oxygen, sulfur, -N(C₁-C₄ alkyl), or CR¹³R¹⁴ wherein R¹³ and R¹⁴ are independently selected from hydrogen, and methyl with the exception that one of R¹³ and R¹⁴ may optionally be cyano;
   with the proviso that: (a) in the six or seven membered rings of structures in formula I, there can not be two double bonds adjacent to each other; and (b) when D is carbon and is double bonded to B, then B is CR¹R²;
   or a pharmaceutically acceptable salt of such compound.
**VII.** The CRF antagonist can also be of the following formula, disclosed in WO 98/08847: 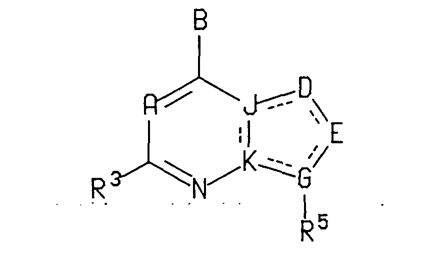 or a pharmaceutically acceptable salt thereof, wherein
   the dashed lines represent optional double bonds;
   A is nitrogen or CR⁷;
   B is -NR¹R², -CR¹R²R¹⁰ -C(=CR²R¹¹)R¹ -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ or -COR²;
   J and K are each independently nitrogen or carbon and both J and K are not nitrogens;
   D and E are each selected, independently, from nitrogen, CR⁴, C=O, C=S, sulfur, oxygen, CR⁴R⁶ and NR⁸;
   G is nitrogen or carbon;
   the ring containing D, E, G, K, and J in formula I may be a saturated or unsaturated 5-membered ring and may optionally contain one or two double bonds and may optionally contain from one to three heteroatoms in the ring and may optionally have one or two C=O or C=S groups;
   R¹ is C₁-C₆ alkyl optionally substituted with one or two substituents independently selected from hydroxy, fluoro, chloro, bromo, iodo, -O-(C₁-C₄ alkyl), CF₃, -C(=O)O-(C₁-C₄alkyl), -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
   R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl) may optionally and independently be replaced by an oxygen or sulfur atom or by NZ² wherein Z² is selected from hydrogen, C₁-C₄ alkyl, benzyl and C₁-C₄ alkanoyl, and wherein each of the foregoing R² groups may optionally be substituted with from one to three substituents independently selected from chloro, fluoro, hydroxy and C₁-C₄ alkyl, or with one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR¹R² or CR¹R²R¹⁰ may form a saturated 3 to 8 membered carbocyclic ring which may optionally contain from one to three double bonds and wherein one or two of the ring carbon atoms of such 5 to 8 membered rings may optionally and independently be replaced by an oxygen or sulfur atom or by NZ³ wherein Z³ is hydrogen, C₁-C₄ alkyl, benzyl or C₁-C₄ alkanoyl;
   R³ is hydrogen, C₁-C₄ alkyl, -O(C₁-C₄ alkyl), chloro, fluoro, bromo, iodo, (C₁-C₂ alkylene)-O-(C₁-C₂ alkyl), (C₁-C₂ alkylene)-OH, or -S(C₁-C₄ alkyl);
   each R⁴ is, independently, hydrogen, (C₁-C₆ alkyl), fluoro, chloro, bromo, iodo, hydroxy, cyano, amino, (C₁-C₂ alkylene)-OH, CF₃, CH₂SCH₃, nitro, -O(C₁-C₄ alkyl), -N(C₁-C₄ alkyl)(C₁-C₂ alkyl), -S(C₁-C₄ alkyl), -CO(C₁-C₄ alkyl), -C(=O)H or -C(=O)O(C₁-C₄alkyl);
   R⁶ is hydrogen, methyl or ethyl;
   R⁸ is hydrogen or C₁-C₄ alkyl;
   R⁵ is phenyl, pyridyl, pyrazinyl, pyrimidyl, pyridazinyl and wherein each of the foregoing R⁵ groups is substituted with from one to four substituents R¹³ wherein one to three of said substituents may be selected, independently, from fluoro, chloro, C₁-C₆ alkyl and -O(C₁-C₆ alkyl) and one of said substituents may be selected from bromo, iodo, formyl, OH, (C₁-C₄ alkylene)-OH, (C₁-C₄alkylene)-O-(C₁-C₂ alkyl), -CN, -CF₃, -NO₂, -NH₂, -NH(C₁-C₄ alkyl), -N(C₁-C₂ alkyl)(C₁-C₆ alkyl), -OCO(C₁-C₄ alkyl), (C₁-C₄ alkylene)-O-(C₁-C₄ alkyl), -S(C₁-C₆ alkyl), (C₁-C₄ alkylene)-S-(C₁-C₄ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R⁵ groups may optionally have one or two double bonds;
   R⁷ is hydrogen, C₁-C₄ alkyl, halo (e.g., chloro, fluoro, iodo or bromo), hydroxy, -O(C₁-C₄ alkyl), -C(=O)(C₁-C₄ alkyl), -C(=O)O(C₁-C₄ alkyl), -OCF₃, -CF₃, -CH₂OH or -CH₂O(C₁-C₂ alkyl);
   R¹⁰ is hydrogen, hydroxy, methoxy or fluoro;
   R¹¹ is hydrogen or C₁-C₄ alkyl; and
   with the proviso that: a) when both J and K are carbons and D is CR⁴ and E is nitrogen, then G can not be nitrogen; (b) when both J and K are carbons and D and G are nitrogens, then E can not be CR⁴ or C=O or C=S; (c) when both J and K are carbons and D and E are carbons, then G can not be nitrogen; (d) when G is carbon, it must be double banded to E; and (e) in the ring containing J, K, D, E and G, there can not be two double bonds adjacent to each other;
   and the pharmaceutically acceptable salts of such compounds.
**VIII.** Other useful CRF antagonists are of the following formula, disclosed in WO 98/08846: 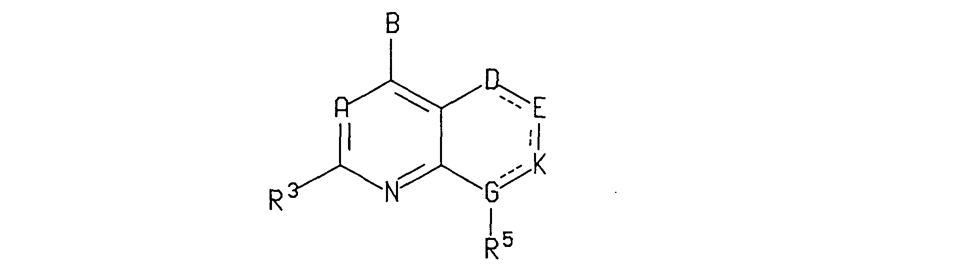 wherein the dashed lines represent optional double bonds;
   A is nitrogen or CR⁷;
   B is -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR¹⁰R¹⁰SR¹ or-COR²;
   G is nitrogen or CR⁴ and is single bonded to all atoms to which it is attached, or G is carbon and is double bonded to K;
   K is nitrogen or CR⁶ when double bonded to G or E, or K is oxygen, sulfur, C=O, C=S, CR⁶R¹² or NR⁸ when single bonded to both adjacent ring atoms, or K is a two atom spacer, wherein one of the two ring atoms of the spacer is oxygen, nitrogen, sulfur, C=O, C=S, CR⁶R¹², NR⁶ or CR⁶, and the other is CR⁶R¹² or CR⁹;
   D and E are each, independently, C=O, C=S, sulfur, oxygen, CR⁴R⁶ or NR⁸ when single bonded to both adjacent ring atoms, or nitrogen or CR⁴ when it is double bonded to an adjacent ring atom;
   the 6- or 7-membered ring that contains D, E, K and G may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
   R¹is C₁-C₆ alkyl optionally substituted with from one or two substituents independently selected from hydroxy, fluoro, chloro, bromo, iodo, C₁-C₄ alkoxy, CF_{3,} -C(=O)(C₁-C₄alkyl), -C(=O)-O-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
   R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl may optionally and independently be replaced by an oxygen or sulfur atom or by NZ wherein Z is hydrogen, C₁-C₄ alkyl or benzyl, and wherein each of the foregoing R² groups may optionally be substituted with from one to three substituents independently selected from chloro, fluoro, hydroxy and C₁-C₄ alkyl, or with one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO_{2,} -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR¹R² or CR¹R²R¹⁰ may form a ring selected from saturated 3 to 8 membered rings, the 5 to 8 membered rings of which may optionally contain one or two double bonds, and wherein one or two of the ring carbon atoms of such 5 to 8 membered rings may optionally and independently be replaced by an oxygen or sulfur atom or by NZ² wherein Z² is hydrogen, benzyl or C₁-C₄ alkyl;
   R³ is hydrogen, C₁-C₄ alkyl, -O(C₁-C₄ alkyl), chloro, fluoro, bromo, iodo, -S(C₁-C₄ alkyl) or -SO₂(C₁-C₄ alkyl);
   each R⁸, R⁹ and R¹² is selected, independently, from hydrogen and C₁-C₂ alkyl;
   each R⁴ and R⁶ that is attached to a carbon atom is selected, independently, from hydrogen and C₁-C₆ alkyl, fluoro, chloro, bromo, iodo, hydroxy, hydroxy (C₁-C₂ alkyl), trifluoromethyl, cyano, amino, nitro, -O(C₁-C₄ alkyl), -N(C₁-C₄ alkyl)(C₁-C₂ alkyl), -CH₂SCH₃, -S(C₁-C₄ alkyl), -CO(C₁-C₄ alkyl), -C(=O)H or -C(=O)O(C₁-C₄ alkyl), wherein each of the C₁-C₂ alkyl moieties in the foregoing R⁴ and R⁶ groups may optionally contain one double or triple bond; and R⁶, when attached to a nitrogen atom, is selected from hydrogen and C₁-C₄ alkyl;
   R⁵ is substituted phenyl, naphthyl, pyridyl or pyrimidyl, wherein each of the foregoing R⁵ groups is substituted with from two to four substituents R¹³, wherein up to three of said substituents may be selected, independently, from chloro, C₁-C₆ alkyl, -O(C₁-C₆ alkyl) and -(C₁-C₆ alkylene)O(C₁-C₆alkyl), and wherein one of said substituents may be selected, independently, from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -(C₀-C₁alkylene)-S-(C₁-C₂alkyl), -(C₀-C₁alkylene)-SO-(C₁-C₂alkyl), -(C₀-C₁alkylene)-SO₂-(C₁-C₂alkyl) and -(C₁-C₄alkylene)-OH, and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
   R⁷ is hydrogen, methyl, halo (e.g., chloro, fluoro, iodo or bromo), hydroxy, methoxy, -C(=O)(C₁-C₂ alkyl), -C(=O)O(C₁-C₂ alkyl), hydroxymethyl, trifluoromethyl or formyl;
   R¹⁰ is hydrogen, hydroxy, methoxy or fluoro; and
   R¹¹ is hydrogen or C₁-C₄ alkyl;
   with the proviso that in the ring containing D, E, K and G of formula I, there can not be two double bonds adjacent to each other;
   and the pharmaceutically acceptable salt of such compound.
**IX.** The CRF antagonist may also be of the following formula, disclosed in WO 95/10506: 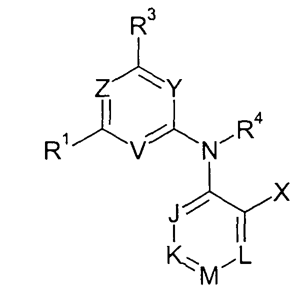 or a pharmaceutically, acceptable salt or prodrug thereof, wherein Y is CR^{3a}, N, or CR²⁹;
   when Y is CR^{3a} or N:
      R¹ is independently selected at each occurrence from the group consisting of C₁-C₄ alkyl, C₂-C₄ alkenyl, C₂-C₄ alkynyl, halogen, C₁-C₂ haloalkyl, NR⁶R⁷ ,OR⁸ and S(O)ₙR⁸; R³ is C₁-C₄ alkyl, aryl, C₃-C₆ cycloalkyl, C₁-C₂ haloalkyl, halogen, nitro, NR⁶R⁷, OR⁸, S(O)ₙR⁸ C(=O)R⁹, C(=O)NR⁶R⁷, C(=S)NR⁶R⁷, -(CHR¹⁶)ₖNR⁶R⁷, (CH₂)ₖOR⁸, C(=O)NR¹⁰CH(R¹¹)CO₂R¹², -C(OH)(R²⁵)(R^{25a}), -(CH₂)ₚS(O)ₙ-alkyl, -(CHR¹⁶)R²⁵, -C(CN)(R²⁵)(R¹⁶) provided that R²⁵ is not -NH- containing rings, -C(=O)R²⁵, -CH(CO₂R¹⁶)₂ , NR¹⁰C(=O)CH(R¹¹)NR¹⁰R¹² , NR¹⁰CH(R¹¹)CO₂R¹²; substituted C₁-C₄ alkyl, substituted C₂-C₄ alkenyl, substituted C₂-C₄ alkynyl, substituted C₁-C₄ alkoxy, aryl-(substituted C₁-C₄) alkyl, aryl-(substituted C₁-C₄) alkoxy, substituted C₃-C₆ cycloalkyl, amino-(substituted C₁-C₄)alkyl, substituted C₁-C₄ alkylamino, where substitution by R²⁷ can occur on any carbon containing substituent; 2-pyridinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, phenyl, -1*H*-indazolyl, 2-pyrrolidonyl, 2*H*,6*H*-1,5,2-dithiazinyl, 2*H*-pyrrolyl, 3*H*-indolyl, 4-piperidonyl, 4a*H*-carbazolyl, 4*H*-quinolizinyl, 6*H*-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, imidazolidinyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenoxathiinyl, phenoxazinyl, phthalazinyl, piperazinyl, piperidinyl, pteridinyl, purinyl, pyranyl, pyrazolidinyl, pyrazolinyl, pyrazolyl, pyridazinyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrahydroisoquinolinyl, tetrahydroquinolinyl, tetrazolyl, thianthrenyl. thiazolyl, thiophenyl, triazinyl, xanthenyl; or 1-tetrahydroquinolinyl or 2-tetrahydroisoquinolinyl either of which can be substituted with 0-3 groups chosen from keto and C₁-C₄ alkyl; J, K, and L are independently selected at each occurrence from the group of N, CH, and CX';
   M is CR⁵ or N;
   V is CR^{1a} or N;
   Z is CR² or N;
   R^{1a}, R², and R^{3a} are independently selected at each occurrence from the group consisting of hydrogen, halo, halomethyl, C₁-C₃ alkyl, and cyano;
   R⁴ is (CH₂)ₘOR¹⁶, C₁-C₄ alkyl, allyl, propargyl, (CH₂)ₘR¹³, or -(CH₂)ₘOC(O)R¹⁶;
   X is halogen, aryl, heteroaryl, S(O)₂R⁸, SR⁸, halomethyl, -(CH₂)_{P}OR⁸, cyano, -(CHR¹⁶)ₚNR¹⁴R¹⁵, -C(=O)R⁸, C₁-C₆ alkyl, C₄-C₁₀ cycloalkylalkyl, C₁-C₁₀alkenyl, C₂-C₁₀alkynyl, C₂-C₁₀alkoxy, aryl-(C₂-C₁₀)-alkyl, C₃-C₆cycloalkyl, aryl-(C₁-C₁₀)-alkoxy, nitro, thio-(C₁-C₁₀)-alkyl, -C(=NOR¹⁶)-C₁-C₄-alkyl, -C(=NOR¹⁶)H, or -C(=O)NR¹⁴R¹⁵, where substitution by R¹⁸ can occur on any carbon containing substituents;
   X' is independently selected at each occurrence from the group consisting of hydrogen, halogen, aryl, heteroaryl, S(O)ₙR⁸, halomethyl, -(CHR¹⁶)ₚOR⁸, cyano,
   -(CHR¹⁶)ₚNR¹⁴R¹⁵, C(=O)R⁸, C₁-C₆ alkyl, C₂-C₁₀alkenyl, C₂-C₁₀alkynyl, C₁-C₁₀alkoxy, aryl-(C₁-C₁₀)-alkyl, C₃-C₆cycloalkyl, aryl-(C₁-C₁₀)-alkoxy, nitro, thio-(C₁-C₁₀)-alkyl, -C(=NOR¹⁶)-C₁-C₄-alkyl, -C(= NOR¹⁶)H, and -C(=O)NR¹⁴R¹⁵, where substitution by R¹⁶ can occur on any carbon containing substituents;
   R⁵ is halo, -C(=NOR¹⁶)-C₁-C₄-alkyl, C₁-C₄alkyl, C₁-C₃ haloalkyl, -(CHR¹⁶)ₚOR⁸, -(CHR¹⁶)ₚS(O)ₙR⁸, -(CHR⁶)ₚNR¹⁴R¹⁵, C₃-C₆ cycloalkyl, C₂-C₁₀alkenyl, C₂-C₁₀alkynyl, aryl-(C₂-C₁₀)-akyl, aryl-(C₁-C₁₀)-alkoxy, cyano, C₃-C₆ cycloalkoxy, nitro, amino- (C₂-C₁₀)-alkyl, thio-(C₂-C₁₀)-alkyl, SOₙ(R⁸), C(=O)R⁸ -C(=NOR¹⁶)H, or -C(=O)NR¹⁴R¹⁵, where substitution by R¹⁸ can occur on any carbon containing substituents;
   R⁶ and R⁷ are independently selected at each occurrence from the group consisting of hydrogen, C₁-C₆ alkyl, C₃-C₁₀ cycloalkyl, C₁-C₆ alkoxy, (C₄-C₁₂)-cycloalkylalkyl, -(CH₂)ₖR¹³. (CHR¹⁶)_{P}OR⁸, -(C₁-C₆alkyl)-aryl, heteroaryl, -S(O)_{z}-aryl or -(C₁-C₆alkyl)-heteroaryl or aryl, wherein the aryl or heteroaryl groups are optionally substituted with 1-3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), cyano, and S(O)₂-(C₁-C₆-alkyl); or can be taken together to form -(CH₂)ₚA(CH₂)ᵣ- , optionally substituted with 0-3 R¹⁷; or, when considered with the commonly attached nitrogen, can be taken together to form a heterocycle, said heterocycle being substituted on carbon with 1-3 groups consisting of hydrogen, C₁-C₆ alkyl, hydroxy, or C₁-C₆ alkoxy;
   A is CH_{2,} O, NR²⁵, C(=O), S(O)ₙ, N(C(=O)R¹⁷), N(R¹⁹), C(H)(NR¹⁴R¹⁵), C(H)(OR²⁰), C(H)(C(=O)R²¹), or N(S(O)ₙR²¹);
   R⁸ is independently selected at each occurrence from the group consisting of hydrogen; C₁-C₆ alkyl; -(C₄-C₁₂) cycloalkylalkyl; (CH₂)ₜR²²; C₃-C₁₀ cycloalkyl; -NR⁶R⁷; aryl; heteroaryl; -NR¹⁶(CH₂)ₙR⁶R⁷; -(CH₂)ₖR²⁵ ; and (CH₂)ₜheteroaryl or (CH₂)ₜaryl, either of which can optionally be substituted with 1-3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), cyano, and S(O)₂(C₁-C₆-alkyl);
   R⁹ is independently selected at each occurrence from R¹⁰, hydroxy, C₁-C₄ alkoxy, C₃-C₆ cycloalkyl, C₂-C₄ alkenyl, aryl substituted with 0-3 R¹⁸, and -(C₁-C₆ alkyl)-aryl substituted with 0-3 R¹⁸;
   R¹⁰, R¹⁶, R²⁴, and R² are independently selected at each occurrence from hydrogen or C₁-C₄ alkyl;
   R¹¹ is C₁-C₄ alkyl substituted with 0-3 groups chosen from the following: keto, amino, sulfhydryl, hydroxyl, guanidinyl, p-hydroxyphenyl, imidazolyl, phenyl, indolyl, and indolinyl, or, when taken together with an adjacent R¹⁰, are (CH₂)ₜ;
   R¹² is hydrogen or an appropriate amine protecting group for nitrogen or an appropriate carboxylic acid protecting group for carboxyl;
   R¹³ is independently selected at each occurrence from the group consisting of CN, OR¹⁹, SR¹⁹, and C₃-C₆ cycloalkyl;
   R¹⁴ and R¹⁵ are independently selected at each occurrence from the group consisting of hydrogen, C₄-C₁₀, cycloalkyl-alkyl, and R₁₉;
   R¹⁷ is independently selected at each occurrence from the group consisting of R¹⁰, C₁-C₄ alkoxy, halo, OR²³, SR²³, NR²³R²⁴, and (C₁-C₆) alkyl (C₁-C₄) alkoxy;
   R₁₈ is independently selected at each occurrence from the group consisting of R¹⁰, hydroxy, halogen, C₁-C₂ haloalkyl, C₁-C₄ alkoxy, C(=O)R²⁴, and cyano;
   R¹⁹ is independently selected at each occurrence from the group consisting of C₁-C₆ alkyl, C₃-C₆ cycloalkyl, (CH₂)_{w}R²² , and aryl substituted with 0-3 R¹⁸;
   R²⁰ is independently selected at each occurrence from the group consisting of R¹⁰, C(=O)R³¹ , and C₂-C₄ alkenyl;
   R²¹ is independently selected at each occurrence from the group consisting of R¹⁰, C₁-C₄ alkoxy, NR²³R²⁴, and hydroxyl;
   R²² is independently selected at each occurrence from the group consisting of cyano, OR²⁴, SR²⁴, NR²³R²⁴,C₁-C₆ alkyl, C₃-C₆ cycloalkyl, -S(O)ₙR³¹, and -C(=O)R²⁵;
   R²⁵, which can be optionally substituted with 0-3 R17, is independently selected at each occurrence from the group consisting of phenyl, pyrazolyl, imidazolyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, 1*H*-indazolyl, 2-pyrrolidonyl, 2*H*,6*H*-1,5,2-dithiazinyl, 2H-pyrrolyl, 3H-indolyl, 4-piperidonyl, 4a*H*-carbazolyl, 4*H*-quinolizinyl, 6*H*-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, indolinyl, indolizinyl, indolyl, isobenzofuranyl, isochromanyl, isoindolinyl, isoindolyl, isoquinolinyl benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazolidinyl, pyridazinyl, pyridyl, pyrimidinyl, pyrrolidinyl, pyrrolinyl, pyrrolyl, quinazolinyl, quinolinyl, quinoxalinyl, quinuclidinyl, B-carbolinyl, tetrahydrofuranyl, tetrazolyl, thianthrenyl, thiazolyl, thiophenyl, triazinyl, xanthenyl; and 1 - tetrahydroquinolinyl or 2-tetrahydroisoquinolinyl either of which can be substituted with 0-3 groups chosen from keto and C₁-C₄ alkyl;
   R^{25a}, which can be optionally substituted with 0-3 R¹⁷ is independently selected at each occurrence from the group consisting of H and R²⁵;
   R²⁷ is independently selected at each occurrence from the group consisting of C₁-C₃ alkyl, C₂-C₄ alkenyl, C₂-C₄ alkynyl, C₂-C₄ alkoxy, aryl, nitro, cyano, halogen, aryloxy, and heterocycle optionally linked through 0;
   R³¹ is independently selected at each occurrence from the group consisting of C₁-C₄ alkyl, C₃-C₇ cycloalkyl, C₄-C₁₀ cycloalkyl-alkyl, and aryl-(C₁-C₄) alkyl;
   k, m, and r are independently selected at each occurrence from 1-4;
   n is independently, selected at each occurrence from 0-2,
   p, q, and z are independently selected at each occurrence from 0-3;
   t and w are independently selected at each occurrence from 1-6,
      provided that when J is CX' and K and L are both CH, and M is CR⁵, then
      (A) when V and Y are N and Z is CH and R¹ and R³ are methyl,
         (1) and R⁴ is methyl, then
            (a) R⁵ can not be methyl when X is OH and X' is H;
            (b) R⁵ can not be -NHCH₃, or -N(CH₃)₂ when X and X' are -OCH₃; and
            (c) R⁵ can not be -N(CH₃)₂ when X and X' are -OCH₂CH₃;
         (2) and R⁴ is ethyl, then
            (a) R⁵ can not be methylamine when X and X' are -OCH₃;
            (b) R⁵ can not be OH when X is Br and X' is OH; and
            (c) R⁵ can not be -CH₂OH or -CH₂N(CH₃)₂ when X is -SCH₃ and X' is H;
      (B) when V and Y are N, Z is CH, R⁴ is ethyl, R⁵ is iso-propyl, X is Br, X' is H, and
         (1) R¹ is CH_{3,} then
            (a) R³ can not be OH, piperazin-1-yl, -CH_{2,}-piperidin-1-yl, -CH₂-(N-4-methylpiperazi n-1-yl), -C(O)NH-phenyl, -CO₂H, -CH₂O-(4-pyridyl), -C(O)NH₂, 2-indolyl, -CH₂O-(4-carboxyphenyl), -N(CH₂CH₃)(2-bromo-4-isopropylphenyl);
         (2) R² is -CH₂CH₂CH₃ then R³ can not be -CH₂CH₂CH₃
      (C) when V, Y and Z are N, R⁴ is ethyl, and
         (1) R⁵ is iso-propyl, X is bromo, and X' is H, then
            (a) R³ can not be OH or -OCH₂CN when R¹ is CH₃ and
            (b) R³ can not be -N(CH₃)₂ when R¹ is -N(CH₃)₂;
         (2) R⁵ is -OCH_{3,} X is -OCH₃, and X' is H, then R³ and R¹ can not both be chloro; further provided that when J, K, and L are all CH and M is CR⁵, then
      (D) at least one of V, Y, and Z must be N;
      (E) when V is CR^{1a}, Z and Y can not both be N;
      (F) when Y is CR^{3a}, Z and V can not both be N;
      (G) when Z is CR², V and Y must both be N;
      (H) Z can be N only when both V and Y are N or when V is CR^{1a} and Y is CR^{3a};
      (I) when V and Y are N, Z is CR², and R² is H or C₁-C₃ alkyl, and R⁴ is C₁-C₃ alkyl, R³ can not be 2-pyridinyl, indolyl, indolinyl, imidazolyl, 3-pyridinyl, 4-pyridinyl, 2-rnethyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-phenothiazinyl, or 4-pyrazinyl;
      (J) when V and Y are N; Z is CR²; R² is H or C₁-C₃ alkyl; R⁴ is C₁-C₄ alkyl, R⁵, X, and/or X' are OH, halo, CF₃, C₁-C₄ alkyl, C₁-C₄ alkoxy, C₁-C₄ alkylthio, cyano, amino, carbamoyl, or C₁-C₄ alkanoyl; and R¹ is C₁-C₄ alkyl, then R⁴ can not be -NH(substituted phenyl) or -N(C₁-C₄ alkyl) (substituted phenyl);
      and wherein, when Y is CR²⁹:
   J, K, L, M, Z, A, k, m, n, p, q, r, t, w, R³, R¹⁰, R¹¹, R¹², R¹³, R¹⁶, R¹⁸, R¹⁹, R²¹, R²³, R²⁴, R²⁵, and R²⁷ are as defined above and R^{25a}, in addition to being as defined above, can also be C₁-C₄ alkyl, but
   V is N;
   R¹ is C₁-C₂ alkyl, C₂-C₄ alkenyl, C₂-C₄ alkynyl, C₂-C₄ alkoxy, halogen, amino, methylamino, dimethylamino, aminomethyl, or N-methylaminomethyl;
   R² is independently selected at each occurrence from the group consisting of hydrogen, halo, C₁-C₃, alkyl, nitro, amino, and -CO₂R¹⁰;
   R₄ is taken together with R²⁹ to form a 5-membered ring and is -C(R²⁶) = or -N= when R²⁹ is -C(R³⁰)= or -N=, or -CH(R²⁶)- when R²⁹ is -CH(R³⁰)-;
   X is Cl, Br, I, S(O)nR⁸, OR⁸, halomethyl, -(CHR¹⁶)ₚOR⁸, cyano, -(CHR¹⁶)ₚNR¹⁴R¹⁵, C(=O)R⁸, C₁-C₆ alkyl, C₂-C₁₀ alkenyl, C₂-C₁₀ alkynyl, C₁-C_{10,} alkoxy, aryl-(C₁-C₁₀)-alkyl, C₃-C₆ cycloalkyl, aryl-(C₁-C₁₀)-alkoxy, nitro, thio-(C₁-C₁₀)-alkyl, -C(=NOR¹⁶)-C₁-C₄-alkyl, -C(=NOR¹⁶)H, or C(=O)NR¹⁴R¹⁵ where substitution by R¹⁸ can occur on any carbon containing substituents;
   X' is hydrogen, CI, Br, I, S(O)ₙR⁸, -(CHR¹⁶)ₚOR⁸, halomethyl, cyano, -(CHR¹⁶)ₚNR¹⁴R¹⁵, C(=O)R⁸, C₁-C₆ alkyl, C₂-C₁₀alkenyl, C₂-C₁₀, alkynyl, C₁-C₁₀ alkoxy, aryl-(C₁-C₁₀)-alkyl, C₃-C₆ cycloalkyl, aryl-(C₂-C₁₀)-alkoxy, nitro, thio-(C₂-C₁₀)-alkyl, -C(=NOR¹⁶)-C₁-C₄-alkyl, -C(=NOR¹⁶)H, or C(=O)NR⁸R¹⁵ where substitution by R¹⁸ can occur on any carbon containing substituents;
   R⁵ is halo, -C(=NOR¹⁶)-C₁-C₄-alkyl, C₁-C₆ alkyl, C₁-C₃ haloalkyl, C₁-C₆ alkoxy, (CHR¹⁶)ₚOR⁵, (CHR¹⁶)_{P}S(O)ₙR⁸, (CHR¹⁶)ₚNR¹⁴R¹⁵, C₃-C₆ cycloalkyl, C₂-C₁₀ alkenyl, C₂-C₁₀ alkynyl, aryl-(C₂-C₁₀)-alkyl, aryl-(C₁-C₁₀)-alkoxy, cyano, C₃-C₆ cycloalkoxy, nitro, amino-(C₁-C₁₀)-alkyl, thio-(C₁-C₁₀)-alkyl, SOₙ(R⁸), C(=O)R⁸, -C(=NOR¹⁶)H, or C(=O)NR⁸R¹⁵ where substitution by R¹⁸ can occur on any carbon containing substituents;
   R⁶ and R⁷ are independently selected at each occurrence from the group consisting of hydrogen, C₁-C₆ alkyl, C₃-C₁₀) cycloalkyl, -(CH₂)ₖR¹³, (C₄-C₁₂)-cycloalkylalkyl, C₁-C₆ alkoxy, -(C₁-C₆ alkyl)-aryl, heteroaryl, aryl, -S(O)_{z}-ary) or -(C₁-C₆ alkyl)-heteroaryl or aryl wherein the aryl or heteroaryl groups are optionally substituted with 1-3 groups selected from hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), and cyano; or can be taken together to form -(CH₂)_{q}A(CH₂)ᵣ-, optionally substituted with 0-3 R¹⁷; or, when considered with the commonly attached nitrogen, can be taken together to form a heterocycle, said heterocycle being substituted on carbon with 1-3 groups consisting of hydrogen, C₁-C₆ alkyl, hydroxy, or C₁-C₆ alkoxy;
   R⁸ is independently selected at each occurrence from the group consisting of hydrogen, C₁-C₆ alkyl, -(C₄-C₁₂) cycloalkylalkyl, (CH₂)ₜR²², C₃-C₁₀ cycloalkyl, -(C₁-C₆ alkyl)-aryl, heteroaryl, -NR¹⁶, -N(CH₂)ₙNR⁶R⁷; -(CH₂)ₖR²⁵, -(C₁-C₆ alkyl)-heteroaryl or aryl optionally substituted with 1-3 groups selected from hydrogen, halogen, C₁-C₆ alkyl, C₁-C₆ alkoxy, amino, NHC(=O)(C₁-C₆ alkyl), NH(C₁-C₆ alkyl), N(C₁-C₆alkyl)₂, nitro, carboxy, CO₂(C₁-C₆ alkyl), and cyano;
   R⁹ is independently selected at each occurrence from R¹⁰, hydroxy, C₁-C₄ alkoxy, C₃-C₆ cycloalkyl, C₂-C₄ alkenyl, and aryl substituted with 0-3 R¹⁸;
   R¹⁴ and R¹⁵ are independently selected at each occurrence from the group consisting of hydrogen, C₁-C₆ alkyl, C₃-C₆ cycloalkyl, (CH₂)ₜR²², and aryl substituted with 0-3 R¹⁸;
   R¹⁷ is independently selected at each occurrence from the group consisting of R¹⁰, C₁-C₄ alkoxy, halo, OR²³, SR²³, and NR²³R²⁴;
   R²⁰ is independently selected at each occurrence from the group consisting of R¹⁰ and C(=O)R³¹;
   R²² is independently selected at each occurrence from the group consisting of cyano, OR²⁴, SR²⁴, NR²³R²⁴, C₃-C₆ cycloalkyl, -S(O)ₙR³¹, and -C(=O)R²⁵;
   R²⁶ is hydrogen or halogen;
   R²⁸ is C₁-C₂, alkyl, C₂-C₄ alkenyl, C₂-C₄ alkynyl, hydrogen, C₁-C₂ alkoxy, halogen, or C₂-C₄ alkylamino;
   R²⁹ is taken together with R⁴ to form a five membered ring and is: -CH(R³⁰)- when R⁴ is -CH(R²⁸)-, -C(R³⁰) = or -N = when R⁴ is -C(R²⁸) = or -N=;
   R³⁰ is hydrogen, cyano, C₁-C₂ alkyl, C₁-C₂ alkoxy, halogen, C₁-C₂ alkenyl, nitro, amido, carboxy, or amino;
   R³¹ is C₁-C₄ alkyl, C₃-C₇ cycloalkyl, or aryl-(C₁-C₄) alkyl; provided that when J, K, and L are all CH, M is CR⁵, Z is CH, R³ is CH₃, R²⁸ is H, R⁵ is isopropyl, X is Br, X' is H, and R¹ is CH₃, then R³⁰ can not be H, -CO₂H, or -CH₂NH₂; and further provided that when J, K and L are all CH; M is CR⁵; Z is N; and
      (A) R²⁹ is -C(R³⁰)=; then one of R²⁸ or R³⁰ is hydrogen;
      (B) R²⁹ is N; then R³ is not halo, NH₂, NO₂, CF₃, CO₂H, CO₂-alkyl, alkyl, acyl, alkoxy, OH, or -(CH₂)ₘOalkyl;
      (C) R²⁹ is N; then R²⁸ is not methyl if X or X' are bromo or methyl and R⁵ is nitro; or
      (D) R²⁹ is N; and R¹ is CH₃; and R³ is amino; then R⁵ is not halogen or methyl.

   Preferred compounds of this group include those wherein:
   i) V is N, R¹ is methyl; and R³ is aryl, NR⁶R⁷, or OR⁸;
   ii) V is N, R¹ is methyl; R³ is aryl, NR⁶R⁷, or OR⁸; and R⁴ is methyl or ethyl;
   iii) V is N, R¹ is methyl; R³ is aryl, NR⁶R ⁷, or OR⁸; R⁴ is methyl or ethyl; and X is O(C₁-C₄ alkyl), Br, or C₁-C₄ alkyl;
   iv) V is N, R¹ is methyl; R³ is aryl, NR⁶R⁷, or OR⁸; R⁴ is methyl, ethyl; X is OMe, Br, or (C₁-C₄ alkyl), M is C₁-C₄ alkyl, Br, CI, or O(C₁-C₄ alkyl); and
   v) V is N, R¹ is methyl; R³ is aryl, NR⁶R⁷, OR⁸; or R⁴ is methyl, ethyl; X is OMe, Br, or C₁-C₄ alkyl, M is C₁-C₄ alkyl, Br, CI, or O(C₁-C₄ alkyl); and L is CH, or N.
**X.** The invention also encompasses use of aminothiazole derivatives of the following formula, disclosed in WO 97/00868: 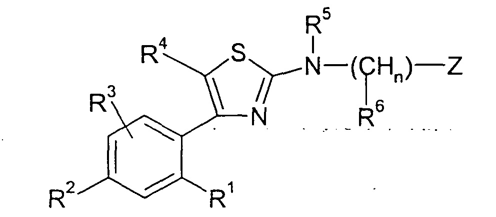 wherein each of R¹ and R² is independently a halogen atom; a C₁-C₅ hydroxyalkyl radical; C₁-C₅ alkyl; C₇-C₁₀ aralkyl; C₁-C₅ alkoxy; trifluoromethyl; nitro; nitrile; a group -SR where R is hydrogen, a C₁-C₅ alkyl radical or a C₇-C₁₀ aralkyl radical; a group S-CO-R where R is a C₁-C₅ alkyl radical or aralkyl in which the aryl portion is C₆-C₈ and the alkyl portion is C₁-C₄; a group -COOR' where R' is hydrogen or C₁-C₅ alkyl; a group -CONR'R" where R' and R" are as defined above for R'; a group -NR'R" where R' and R" are as previously defined for R'; a group -CONRaRb or NRaRb, where Ra and Rb, taken together with the nitrogen atom to which they are attached, form a 5- to 7-membered heterocyclic ring; or a group -NHCO-NR'R", where R' and R" are as defined above for R'; R³ is hydrogen or as defined for R¹ and R² is a hydrogen atom; C₁₋₅ alkyl; halogen; a hydroxymethyl group; or a formyl group; R⁵ is C₁-C₅ alkyl; a C₃-C₇ cycloalkyl group; a cycloalkylalkyl group in which the cycloalkyl portion is C₃-C₇ and the alkyl portion is C₁-C₅; or C₅-C₆ alkenyl; n is 0 or 1; R⁶ is C₁₋₅ alkyl; alkoxyalkyl in which the alkyl portions are C₁-C₅; C₃-C₇ cycloalkyl; a cycloalkylalkyl group in which the cycloalkyl portion is C₃-C₇ and the alkyl portion is C₁-C₅; a cycloalkyloxyalkyl radical in which the cycloalkyl is C₃-C₇ and the alkyl is C₁-C₄; a hydroxyalkyloxyalkyl radical in which the alkyls are C₂-C₁₀; or an alkoxyalkyloxyalkyl radical in which the alkyls are C₃-C₁₂; and Z is an optionally substituted bi- or tricyclic aromatic or heteroaromatic group; and stereoisomers and/or addition salts thereof.
**XI**. CRF antagonists of the following formula, disclosed in WO 97/29109, may also be employed: 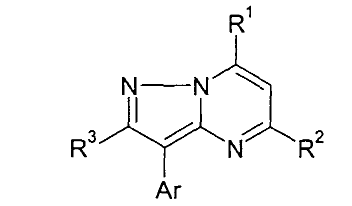 including the stereoisomers and the pharmaceutically acceptable acid addition salt forms thereof, wherein
   R¹ is NR⁴R⁵ or OR⁵;
   R² is C₁-C₆alkyl, C₁-C₆alkyloxy or C₁-C₆alkylthio,
   R³ is hydrogen, C₁-C₆alkyl, C₁-C₆alkylsulfonyl, C₁-C₆alkylsulfoxy or C₁-C₆alkylthio;
   R⁴ is hydrogen, C₁-C₆alkyl, mono- or di(C₃-C₆cyloalkylmethyl, C₃-C₆cyloalkyl, C₃-C₆alkenyl, hydroxyC₁-C₆alkyl, C₁-C₆akylcarbonyloxyC₁-C₆alkyl or C₁-C₆alkyloxyC₁-C₆alkyl;
   R⁵ is C₁-C₈alkyl, mono- or di(C₃-C₆cycloalkyl)methyl, Ar¹CH₂, C₃-C₆alkenyl, C₁-C₆alkyloxyC₁-C₆alkyl, hydroxyC₁-C₆alkyl, thienylmethyl, furanylmethyl, C₁-C₆alkylthioC₁-C₆alkyl, morpholinyl, mono- or di(C₁-C₆alkyl)aminoC₁₋₆alkyl, di(C₁-C₆alkyl)amino, C₁-C₆alkylcarbonylC₁-C₆alkyl, C₁-C₆alkyl substituted with imidazolyl; or a radical of formula -Alk-O-CO-Ar¹;
   or R⁴ and R⁵ taken together with the nitrogen atom to which they are attached may form a pyrrolidinyl, piperidinyl, homopiperidinyl or morpholinyl group, optionally substituted with C₁-C₆alkyl or C₁-C₆alkyloxyC₁-C₆alkyl; and
   Ar is phenyl; phenyl substituted with 1, 2 or 3 substituents independently selected from halo, C₁-C₆alkyl, trifluoromethyl, hydroxy, cyano, C₁-C₆alkyloxy, benzyloxy, C₁-C₆alkylthio, nitro, amino and mono- or di(C₁-C₆alkyl)amino; pyridinyl; pyridinyl substituted with 1 ~ 2 or 3 substituents independently selected from halo, C₁-C₆alkyl, trifluoromethyl, hydroxy, cyano, C₁-C₆alkyloxy, benzyloxy, C₁-C₆alkylthio, nitro, amino, mono- or di(C₁-C₆alkyl)amino and piperidinyl; and wherein said substituted phenyl may optionally be further substituted with one or more halogens;
   Ar¹ is phenyl; phenyl substituted with 1, 2 or 3 substituents each independently selected from halo, C₁-C₆alkyl, C₁-C₆alkyloxy, di(C₁-C₆alkyl)aminoC₁-C₆alkyl, trifluoromethyl and C₁-C₆alkyl substituted with morpholinyl; or pyridinyl; and Alk is C₁-C₆alkanediyl; with the proviso that 5-methyl-3-phenyl-7-(phenylmethoxy)-pyrazolo[1,5-a]-pyrimidine and 2,5-dimethyl-7-(methylamino)-3-phenyl-pyrazolo[1,5-a]pyrimidine are not included.

   Preferred compounds of this formula are those wherein R² is methyl; R³ is hydrogen, or C₁-C₆ alkyl; and Ar is substituted phenyl or 3-pyridyl.
**XII.** CRF antagonists of the following formula, disclosed in WO 97/29110, may also be employed: 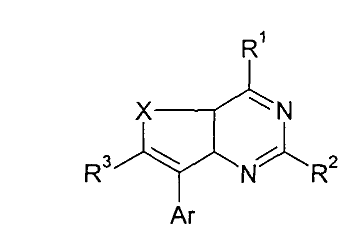 including the stereoisomers and the pharmaceutically acceptable acid addition salt forms thereof, wherein
   X is S. SO or SO₂;
   R¹ is NR⁴R⁵ or OR⁵;
   R² is C₁-C₆alkyl, C₁-C₆alkyloxy or C₁-C₆alkylthio;
   R³ is hydrogen, C₁-C₆alkyl, C₁-C₆alkylsulfonyl, C₁-C₆alkylsulfoxy or C₁-C₆alkylthio;
   R⁴ is hydrogen, C₁₋₆alkyl, mono- or di(C₃-C₆cycloalkyl)methyl, C₃-C₆cycloalkyl, C₃-C₆alkenyl, hydroxyC₁-C₆alkyl, C₁-C₆alkylcarbonylaxyC₁-C₆alkyl or C₁-C₆alkyloxyC₁-C₆alkyl;
   R⁵is C₁-C₈alkyl, mono- or di(C₃-C₆cycloalkyl)methyl, Ar¹CH₂, C₃-C₆alkenyl, C₁-C₆alkyloxyC₁-C₆alkyl, hydroxyC₁-C₆alkyl, thienylmethyl, furanylmethyl, C₁-C₆alkylthioC₁-C₆alkyl, morpholinyl, mono- or di(C₁-C₆alkyl)aminoC₁-C₆alkyl, di(C₁-C₆alkyl)amino, C₁-C₆alkylcarbonylC₁-C₆alkyl, C₁-C₆alkyl substituted with imidazolyl; or a radical of formula -Alk-O-CO-Ar I; or R⁴ and R⁵ taken together with the nitrogen atom to which they are attached may form a pyrrolidinyl, piperidinyl, homopiperidinyl or morpholinyl group, optionally substituted with C₁-C₆alkyl or C₁-C₆alkyloxyC₁-C₆alkyl;
   Ar is phenyl; phenyl substituted with 1, 2 or 3 substituents independently selected from halo, C₁-C₆alkyl, trifluoromethyl, hydroxy, cyano, C₁-C₆alkyloxy, benzyloxy, C₁-C₆alkylthio, nitro, amino and mono- or di(C₁-C₆alkyl)amino; pyridinyl; pyridinyl substituted with 1, 2 or 3 substituents independently selected from halo, C₁-C₆alkyl, trifluoromethyl, hydroxy, cyano, C₁-C₆alkyloxy, benzyloxy, C₁-C₆alkylthio, nitro, amino, mono- or di(C₁-C₆alkyl)amino and piperidinyl; and wherein said substituted phenyl may optionally be further substituted with one or more halogens;
   Ar¹is phenyl; phenyl substituted with 1, 2 or 3 substituents each independently selected from halo, C₁-C₆alkyl, C₁-C₆alkyloxy, di(C₁-C₆alkyl)aminoC₁-C₆alkyl trifluoromethyl, and C₁-C₆alkyl substituted with morpholinyl; or pyridinyl; and
   Alk is C₁-C₆alkanediyl.

Preferred compounds of this group include those wherein:
i) R² is methyl;
ii) R² is methyl; and Ar is substituted phenyl or 3-pyridyl;
iii) R² is methyl; R³ is methyl; and Ar is substituted phenyl or 3-pyridyl.

Specific CRF antagonists useful in the practice of the invention, include, without limitation, the following compounds:
4-(1-ethyl-propoxy)-3,6-dimethyl-2-(2,4,6-trimethylphenoxy)-pyridine;
butyl-[2,5-dimethyl-7-(2,4,6-trimethylphenyl)-6,7-dihydro-5H-pyrrolo[2,3-d]pyrimidin-4-yl]-ethyl-amino;
4-(butyl-ethylamino)-2,5-dimethyl-7-(2,4,6-trimethylphenyl)-5,7-dihydro-pyrrolo[2,3-d]pyrimidin-6-one;
4-(1-ethylpropoxy)-2,5-dimethyl-6-(2,4,6-trimethyiphenoxy)-pyrimidine;
N-butyl-N-ethyl-2,5-dimethyl-NN-(2,4,6-trimethylphenyl)-pyrimidine-4,6-diamine;
[4-(1-ethyl-propoxy)-3,6-dimethyl-pyridin-2-yl]-(2,4,6-trimethylphenyl)-amine;
6-(ethyl-propyl-amino)-2,7-dimethyl-9-(2,4,6-trimethylphenyl)-7,9-dihydro-purin-8-one;
3-{(4-methyl-benzyl)-[3,6-dimethyl-1-(2,4,6-trimethylphenyl)-1H-pyrazolo[3,4-d]pyrimidin-4-yl]-amino}-propan-1-ol;
diethyl-[6-methyl-3-methylsulfanyl-1-(2,4,6-trichlorophenyl)-1H-pyrazolo[3,4-d]pyrimidin-4-yl]-amine;
2-{butyl-(6-methyl-3-methylsulfanyl-1 -(2,4,6-trichlorophenyl)-1H-pyrazolo[3,4-d]pyrimidin-4-yl]-amino}-ethanol;
dibutyl-[6-methyl-3-methylsulfanyl-1-(2,4,6-trichlorophenyl)-1H-pyrazolo[3,4-d]pyrimidin-4-yl}-amine;
butyl-ethyl-[6-methyl-3-methylsulfanyl-1-(2,4,6-trichlorophenyl)-1H-pyrazolo[3,4-d]pyrimidin-4-yl]-amine;
butyl-ethyl-[6-methyl-3-methylsulfonyl-1-(2,4,6-trichlorophenyl)-1H-pyrazolo[3,4-d]pyrimidin-4-yl]-amine;
butyl-cyclopropylmethyl-[6-methyl-3-methylsulfanyl-1-(2,4,6-trichlorophenyl)-1H-pyrazolo[3,4-d]pyrimidin-4-yl]-amine;
di-1-propyl-[6-methyl-3-methylsulfanyl-1-(2,4,6-trichlorophenyl)-1H-pyrazolo[3,4-d]pyrimidin-4-yl]-amine;
diallyl-[6-methyl-3-methylsulfanyl-1-(2,4,6-trichlorophenyl)-1H-pyrazolo[3,4-d]pyrimidin-4-yl]-amine;
butyl-ethyl-[6-chloro-3-methylsulfanyl-1-(2,4,6-trichlorophenyl)-1H-pyrazolo[3,4-d]pyrimidin-4-yl]-amine;
butyl-ethyl-[6-methoxy-3-methylsulfanyl-1-(2,4,6-trichlorophenyl)-1H-pyrazolo[3,4-d]pyrimidin-4-yl]-amine;
propyl-ethyl-[3,6-dimethyl-1-(2,4,6-trimethylphenyl)-1H-pyrazolo[3,4-d]pyrimidin-4-yl]-amine;
4-(1-ethyl-propyl)-6-methyl-3-methylsulfanyl-1-(2,4,6-trimethylphenyl)-1H-pyrazolo[3,4-d]pyrimidine;
n-butyl-ethyl-[2,5-dimethyl-7-(2,4,6-trimethylphenyl)-7H-pyrrolo[2,3-d]pyrimidin-4-yl]amine;
di-n-propyl-[2,5-dimethyl-7-(2,4,6-trimethylphenyl)-7H-pyrrolo[2,3-d]pyrimidin-4-yl]amine;
ethyl-n-propyl-[2,5-dimethyl-7-(2,4,6-trimethylphenyl)-7H-pyrrolo[2,3-d]pyrimidin-4-yl]amine;
diethyl-2,5-dimethyt-7-(2,4,6-trimethylphenyl)-7H-pyrrolo[2,3-d]pyrimidin-4-yl]amine;
n-butyl-ethyl-[2,5,6-trimethyl-7-(2,4,6-trimethylphenyl)-7H-pyrrolo[2,3-d]pyrimidin-4-yl]amine;
2-{N-n-butyl-N-[2,5-dimethyl-7-(2,4,6-trimethylphenyl)-7H-pyrrolo[2,3-d]pyrimidin-4-yl]amino}-ethanol;
4-(1-ethyl-propyl)-2,5,6-trimethyl-7-(2,4,6-trimethylphenyl)-7H-pyrrolo[2,3-d]pyrimidine;
n-butyl-ethyl-[2,5-dimethyl-7-(2,4-dimethylphenyl)-7H-pyrrolo[2,3-d]pyrimidin-4-yl]amine;
2,5-dimethyl-7-(2,4,6-trimethylphenyl)-7H-pyrrolo[2,3-d]pyrimidy1-4-yl]-(1-ethylpropyl)amine;
butyl-[3,6-dimethyl-1-(2,4,6-trimethylphenyl)-1H-pyrazolo[3,4-b]pyridin-4-yl]-ethylamine;
[3,6-dimethyl-1-(2,4,6-trimethylphenyl)-1H-pyrazolo[3,4,b]pyridin-4-yl]-(1-methoxymethylpropyl)-amine;
4-(1-methoxymethylpropoxy)-3,6-dimethyl-1-(2,4,6-trimethylphenyl)-1H-pyrazolo[3,4-b]pyridine;
(1-ethylpropyl)-[3,5,6-trimethyl-1-(2,4,6-trimethylphenyl)-1H-pyrazolo[3,4-b]pyridin-4-yl]-amine;
4-(1-ethylpropoxy)-2,5-dimethyl-7-(2,4,6-trimethylphenyl)-7H-pyrrolo[2,3-b]pyridine;
4-(1-ethylpropoxy)-2,5,6-trimethyl-7-(2,4,6-trimethylphenyl)-7H-pyrrolo[2,3-b]pyridine;
4-(1-ethylpropoxy)-2,5-dimethyl-7-(2,6-dimethyl-4-bromophenyl)-7H-pyrrolo[2,3-b]pyridine;
2,5,6-trimethyl-7-(1-propylbutyl)-4-(2,4,6-trimethylphenoxy)-7H-pyrrolo[2,3-d]pyrimidine;
1-(1-ethylpropyl)-6-methyl-4-(2,4,6-trimethylphenylamino)-1,3-dihydro-imidazo[4,5-c]pyridin-2-one;
9-(1-ethylpropyl)-2-methyl-6-(2,4,6-trimethylphenylamino)-7,9-dihydro-purin-8-one;
1-(1-ethylpropyl)-6-methyl-4-(2,4,6-trimethylphenoxy)-1,3-dihydro-imidazo[4, 5-c]pyridin-2-one;
1-(1-ethylpropyl)-6-methyl-4-(2,4,6-trimethylphenoxy)-1H-imidazo[4,5-c]pyridine;
1-(1-ethylpropy))-3,6-dimethyl-4-(2,4,6-trimethylphenoxy)-1,3-dihydro-imidazo[4,5-c]pyridin-2-one;
1-(1-ethylpropyl)-3,6-dimethyl-4-(2,4,6-trimethylphenylamino)-1,3-dihydro-imidazo[4,5-c]pyridin-2-one;
1-(1-ethyl-propyl)-4,7-d imethyl-5-(2,4,6-trimethyl-phenoxy)-1,4-dihydro-2H-pyrido[3,4-b]pyrazin-3-one;
1-(1-ethy)-propyl)-4,7-dimethyl-5-(2,4,6-trimethy)-phenoxy)-1,4-dihydro-2H-pyrido[3,4-b]pyrazin-3-one;
1-(1-ethyl-propyl)-4,7-dimethyl-5-(2,4,6-trimethyl-phenoxy)-1,2,3,4-tetrahydro-pyrido[3,4-b]pyrazine;
1-(1-ethyl-propyl)-7-methyl-5-(2,4,6-trimethyl-phenoxy)-1,2,3,4-tetrahydro-pyrido[3,4-b]pyrazine;
1-(1-ethyl-propyl)-7-methyl-2-oxo-5-(2,4,6-trimethyl-phenoxy)-1,2,3,4-tetrahydro-[1,6]na phthyridine-3-carboxylic acid methyl ester;
1-(1-ethyl-propyl)-7-methyl-2-oxo-5-(2,4, 6-trimethyl-phenoxy)-1,2,3,4-tetrahydro-[1,6]na phthyridine-3-carboxylic acid isopropyl ester;
1-(1-ethy)-propy))-7-methyl-5-(2,4,6-trimethy)-phenoxy)-3,4-dihydro-1H-[1,6]naphthyridin-2-one;
1-(1-ethyl-propyl)-7-methyl-5-(2,4,6-trimethyl-phenoxy)-1,2,3,4-tetrahydro-[1,6]naphthyridine;
1-(1-ethyl-propyl)-7-methyl-5-(2,4,6-trimethyl-phenoxy)-1,4-dihydro-2H-3-oxa-1,6-diaza-naphthalene;
1-(1-ethyl-propyl)-4,7-dimethyl-5-(2,4,6-trimethyl-phenoxy)-1,4-dihydro-2H-3-oxa-1,6-diaza-naphthalene;
1-(1-ethyl-propyl)-3,7-dimethyl-5-(2,4,6-trimethyl-phenoxy)-3,4-dihydro-1H-3-oxa-[1,6]-naphthyridin-2-one;
1-(1-ethyl-propyl)-3,3,6-trimethyl-4-(2,4,6-trimethyl-phenoxy)-2,3-dihydro-1H-pyrrolo[3,2-c]pyridine;
7-(1-ethyl-propoxy)-5-methyl-3-(2,4,6-trimethyl-phenyl)-pyrazolo[1,5-a]pyrimidine;
[2,5-dimethyl-3-(2,4,6-trimethyl-phenyl)-pyrazolo[1,5-a]pyrimidin-7-yl]-(1-ethyl-propyl)-amine;
(1-ethyl-propyl)-[5-methyl-3-(2,4,6-trimethyl-phenyl)-pyrazolo[1,5-a]pyrimidin-7-yl]-amine;
7-(1-ethyl-propoxy)-2,5-dimethyl-3-(2,4,6-trimethyl-phenyl)-pyrazolo[1,5-a]pyrimidine;
[2,5-dimethyl-3-(2,4, 6-trimethyl-phenyl)-pyrazolo(1,5-a]pyrimidin-7-yl]-ethyl-propyl-amine;
[6-bromo-5-bromomethyl-3-(2,4,6-trimethyl-phenyl)-3H-[1,2,3]triazolo[4,5-b]pyridin-7-yl]-(1-ethyl-propyl)-amine;
(1-ethyl-propyl)-[5-methyl-3-(2,4,6-trimethyl-phenyl)-3H-[1,2,3]triazolo[4,5-b]pyridin-7-yl]-amine;
[6-bromo-5-methyl-3-(2,4,6-trimethyl-phenyl)-3H-[1,2,3]triazolo[4,5-b]pyridin-7-yl]-(1-ethyl-propyl)-methyl-amine;
7-(1-ethyl-propoxy)-5-methyl-3-(2,4,6-trimethyl-phenyl)-3H-[1 ,2,3]triazolo[4,5-b]pyridine;
4-(1-ethyl-propoxy)-2,5-dimethyl-7-(2,4,6-trimethyl-phenyl)-5H-pyrrolo[3,2-d]pyr imidine;
(±)-2,5-dimethyl-4-(tetrahydro-furan-3-yloxy)-7-(2,4,6-trimethyl-phenyl)-5H-pyrrolo-[3,2-d]pyrimidine;
2,5-dimethyl-4-(S)-(tetrahydro-furan-3-yloxy)-7-(2,4,6-trimethyl-phenyl)-5H-pyrrolo-[3,2-d]pyrimidine;
2,5-dimethyl-4-(1-propyl-butoxy)-7-(2,4,6-trimethyl-phenyl)-5H-pyrrolo[3,2-d]pyrimidine;
4-sec-butylsulfanyl-2,5-dimethyl-7-(2,4,6-trimethyl-phenyl)-5H-pyrrolo[3,2-d]pyrimidine;
4-(butyl-ethyl-amino)-2,6-dimethyl-8-(2,4,6-trimethyl-phenyl)-5,8-dihydro-6H-pyrido [2,3-d]pyrimidin-7-one;
8-(1-ethyl-propoxy)-6-methyl-4-(2,4,6-trimethyl-phenyl)-3,4-dihydro-1H- pyrido[2,3-b] pyrazin-2-one;
8-(1-ethyl-propoxy)-6-methyl-4-(2,4,6-trimethyl-phenyl)-1,2,3,4-tetrahydro-pyrido [2,3-b]pyrazine;
4-( 1-ethyl-propoxy)-2-methyl-8-(2,4,6-trimethyl-phenyl)-quinoline;
5-( 1-ethyl-propoxy)-7-methyl-1-(2,4,6-trimethyl-phenyl)-1,4-dihydro-2H-3-oxa-1,8-diaza-naphthalene;
5-(1-ethyl-propoxy)-7-methyl-1-(2.4,6-trimethy-phenyl)-1,2-dihydro.-3-oxa-1,8-diazanaphthalen-4-one;
8-(1-ethyl-propoxy)-1,6-dimethyl-4-(2,4,6-trimethyl-phenyl)-1,2,3,4-tetrahydro-pyrido[2,3-b]pyrazine;
(1-ethyl-propyl)-[2-methyl-8-(2,4,6-trimethyl-phenyl)-quinolin-4-yl]-amine;
4-(butyl-ethyl-amino)-2,6-dimethyl-8-(2,6-dimethyl-4-bromo-phenyl)-5,8-dihydro-6H-pyrido[2,3-d]pyrimidin-7-one;
4-(butyl-ethyl-amino)-2-methyl-8-(2,6-dimethyl-4-bromo-phenyl)-5,8-dihydro-6H-pyrido[2,3-d]pyrimidin-7-one;
4-(1-ethyl-propoxy)-2-methyl-8-(2,6-dimethyl-4-bromo-phenyl)-5,8-dihydro-6H-pyrido[2,3-d]pyrimidin-7-one;
(butyl-ethyl)-[2-methyl-8-(2,6-dimethyl-4-bromo-phenyl)-5,6,7,8-tetrahydro-pyrido[2,3-d] pyrimidin-4-yl]-amine;
(propyl-ethyl)-[2-methyl-8-(2,6-dimethyl-4-bromo-phenyl)-5,6,7,8-tetrahydro-pyrido[2,3-d]pyrimidin-4-yl]-amine;
(diethyl)-[2-methyl-8-(2,6-dimethyl-4-bromo-phenyl)-5,6,7,8-tetrahydro-pyrido [2,3-d]pyrimidin-4-yl]-amine;
(1-ethyl-propyl)-[2-methyl-8-(2,6-dimethyl-4-bromo-phenyl)-5,6,7,8-tetrahydro-pyrido[2,3-d]pyrimidin-4-yl]-amine;
(1-ethyl-propoxy)-2-methyl-8-(2,6-dimethyl-4-bromo-phenyl)-5,6,7,8-tetrahydro-pyrido[2,3-d]pyrimidine;
4-(butyl-ethyl-amino)-2-methyl-8-(2,4,6-trimethyl-phenyl)-5,8-dihydro-6H-pyrido[2,3-d]pyrimidin-7-one;
4-(1-ethyl-propoxy)-2-methyl-8-(2,4,6-trimethyl-phenyl)-5,8-dihydro-6H-pyrido [2,3-d]pyrimidin-7-one;
(butyt-ethyl)-[2-methyl-8-(2,4,6-trimethyl-phenyl)-5,6,7,8-tetrahydro-pyrido[2,3-d] pyrimidin-4-yl]-amine;
(propyl-ethyl)-[2-methyl-8-(2,4,6-trimethyl-phenyl)-5,6,7,8-tetrahydro-pyrido[2,3-d] pyrimidin-4-yl]-amine;
(diethyl)-[2-methyl-8-(2,4,6-trimethyl-phenyl)-5,6,7,8-tetrahydro-pyrido[2,3-d] pyrimidin-4-yl]-amine;
(1-ethyl-propyl)-[2-methyl-8-(2,4,6-trimethyl-phenyl)-5,6,7,8-tetrahydro- pyrido[2,3-d] pyrimidin-4-yl]-amine;
(1-ethyl-propoxy)-2-methyl-8-(2,4,6-trimethyl-phenyl)-5,6,7,8-tetrahydro- pyrido[2,3-d] pyrimidine;
8-(1-ethy)-propoxy)-6-methyl-4-(2,6-dimethyl-4-bromo-phenyl)-3,4-dihydro- 1H-pyrido [2,3-b]pyrazin-2-one;
8-(1-ethyl-propoxy)-6-methyl-4-(2,6-dimethyl-4-bromo-phenyl)-1,2,3,4-tetrahydro-pyrido[2,3-b]pyrazine;
4-(1-ethyl-propoxy)-2-methyl-8-(2,6-dimethyl-4-bromo-phenyl)-quinoline;
5-(1-ethyl-propoxy)-7-methyl-1-(2,6-dimethyl-4-bromo-phenyl)-1,4-dihydro-2H- 3-oxa-1,8-diaza-naphthalene;
5-(1-ethyl-propoxy)-7-methyl-1-(2,6-dimethyl-4-bromo-phenyl)-1,2-dihydro-3-oxa-1, 8-diaza-naphthalen-4-one;
8-(1-ethyl-propoxy)-1,6-dimethyl-4-(2,6-dimethyl-4-bromo-phenyl)-1,2,3,4- tetrahydro-pyrido[2,3-b]pyrazine;
(1-ethyl-propyl)-[2-methyl-8-(2,6-dimethyl-4-bromo-phenyl)-quinolin-4-yl]-amine;
4-(butyl-ethyl-amino)-2,6-dimethyl-8-(2,6-dimethyl-4-chloro-phenyl)-5,8-dihydro-6H-pyrido[2,3-d]pyrimidin-7-one;
8-(1-ethyl-propoxy)-6-methyl-4-(2,6-dimethyl-4-chloro-phenyl)-3,4-dihydro-1H-pyrido[2,3-b]pyrazin-2-one;
8-(1-ethyl-propoxy)-6-methyl-4-(2,6-dimethyl-4-chloro-phenyl)-1,2,3,4-tetrahydro-pyrido[2,3-b]pyrazine;
4-(1-ethyl-propoxy)-2-methyl-8-(2,6-dimethyl-4-chloro-phenyl)-quinoline;
5-(1-ethy(-propoxy)-7-methyl-1-(2,6-dimethyl-4-chloro-phenyl)-1,4-dihydro- 2H-3-oxa-1,8-diaza-naphthalene;
5-(1-ethyl-propoxy)-7-methyl-1-(2,6-dimethyl-4-chloro-phenyl)-1,2-dihydro-3- oxa-1,8-diaza-naphthalen-4-one;
8-(1-ethyl-propoxy)-1,6-dimethyl-4-(2,6-dimethyl-4-chloro-phenyl)-1,2,3,4- tetrahydro-pyrido[2,3-b]pyrazine;
(1-ethyl-propyl)-(2-methyl-8-(2,6-dimethyl-4-chloro-phenyl)-quinolin-4-yl]-amine;
8-(1-hydroxymethyl-propoxy)-6-methyl-4-(2,4,6-trimethyl-phenyl)-3,4-dihydro-1H-pyrido[2,3-b]pyrazin-2-one;
8-(1-hydroxymethyl-propylamino)-6-methyl-4-(2,4,6-trimethyl-phenyl)-3,4- dihydro-1H-pyrido[2,3-b]pyrazin-2-one;
8-(1-ethyl-propylamino)-6-methyl-4-(2,4,6-trimethyl-phenyl)-3,4-dihydro-1H-pyrido[2,3-b]pyrazin-2-one;
8-diethylamino-6-methyl-4-(2,4,6-trimethyl-phenyl)-3,4-dihydro-1H-pyrido[2,3-b] pyrazin-2-one;
8-(ethyl-propyl-amino)-6-methyl-4-(2,4,6-trimethyl-phenyl)-3,4-dihydro-1H-pyrido[2,3-b]pyrazin-2-one;
8-(butyl-ethyl-amino)-6-methyl-4-(2,4,6-trimethyl-phenyl)-3,4-dihydro-1 H-pyrido [2,3-b]pyrazin-2-one;
8-(1-hydroxymethyl-propoxy)-6-methyl-4-(2,4,6-trimethyl-phenyl)-1,2,3,4-tetrahydro-pyrido[2,3-b]pyrazine;
8-(1-hydroxymethy(-propylamino)-6-methyl-4-(2,4,6-trimethyl-phenyl)-1,2,3,4-tetrahydro-pyrido[2,3-b]pyrazine;
8-(1-ethyl-propylamino)-6-methyl-4-(2,4,6-trimethyl-phenyl)-1,2,3,4-tetrahydro-pyrido[2,3-b]pyrazine;
8-diethylamino-6-methyl-4-(2,4,6-trimethyl-phenyl)-1,2,3,4-tetrahydro-pyrido [2,3-b]pyrazine;
8-(ethyl-propyl-amino)-6-methyl-4-(2,4,6-trimethyl-phenyl)-1,2,3,4-tetrahydro-pyrido[2,3-b]pyrazine;
8-(butyl-ethyl-amino)-6-methyl-4-(2,4,6-trimethyl-phenyl)-1,2,3,4-tetrahydro-pyrido[2,3-b]pyrazine;
4-(1-hydroxymethyl-propoxy)-2-methyl-8-(2,4,6-trimethyl-phenyl)-quinoline;
4-(1-hydroxymethyl-propylamino)-2-methyl-8-(2,4,6-trimethyl-phenyl)- quinoline;
4-(1-ethyl-propylamino)-2-methyl-8-(2,4,6-trimethyl-phenyl)- quinoline;
4-diethylamino-2-methyl-8-(2,4,6-trimethyl-phenyl)- quinoline;
4-(ethyl-propyl-amino)-2-methyl-8-(2,4,6-trimethyl-phenyl)- quinoline;
4-(butyl-ethyl-amino)-2-methyl-8-(2,4,6-trimethyl-phenyl)- quinoline;
5-( 1-hydroxymethy)-propoxy)-7-methyl-1-(2,4,6-trimethyl-phenyl)-1,4-dihydro-2H-3-oxa-1,8-diaza-naphthalene;
5-(1-hydroxymethyl-propylamino)-7-methyl-1-(2,4,6-trimethyl-phenyl)-1,4-dihydro-2H-3-oxa-1,8-diaza-naphthalene;
5-(1-ethyl-propylamino)-7-methyl-1-(2,4,6-trimethyl-phenyl)-1,4-dihydro-2H-3-oxa-1,8-diaza-naphthalene;
5-diethylamino-5-methyl-1-(2,4,6-trimethyl-phenyl)-1,4-dihydro-2H-3-oxa-1,8-diaza-naphthalene;
5-(ethyl-propyl-amino)-7-methyl-1-(2,4,6-trimethyl-phenyl)-1,4-dihydro-2H-3-oxa-1,8-diaza-naphthalene;
8-(butyl-ethyl-amino)-6-methyl-4-(2,4,6-trimethyl-phenyl)-1,4-dihydro-2H-3-oxa-1,8-diaza-naphthalene;
4-(2,4-dichlorophenyl)-5-methyl-2-[N-(1-(methoxymethyl)-1-(naphth-2-yl)methyl)-N-pr opylamino]thiazole;
oxalate of 4-(2,4-dichlorophenyl)-5-methyl-2-[N46-methoxyiosoquinol-5-yl)-N-propylamino]thiazole;
oxalate of 4-(2-hloro-4-methoxyphenyl)-5methyl-2[N-(6-methylisoquinol-5-yl)-N-propylamino]thiazole;
4-(2-chloro-4-methoxyphenyl)-5-methyl-2-[N-(1-methoxycarbonylmethylindol-5-yl)-N-p ropylamino]thiazole;
oxalate of 4-(2-chloro-4-methoxyphenyl)-5-methyl-2-[N-(6-methoxy isoquinol-5-yl)-N-propylamino]thiazole;
oxalate of 4-(2-chloro-4-methoxyphenyl)-5-methyl-2-[N-(6-chloroisoquinol-5-yl)-N-propylamino]thiazole;
oxalate of 4-(2-chloro-4-methoxyl)-5-methyl-2-[N-(6-methoxysoquinol-5-yl)-N-propylamino]thiazole;
4-(2-chloro-4-methoxyphenyl)-5-methyl-2-[N-1methoxynaphth-2-yl)-N-propylamino]thiazol;
oxalate of 4-(2-chloro-4-trifluoromethylphenyl)-5-methyl-2-[N-6, methoxyisoquinol-5-yl)-N-propylamino]thiazole;
chlorhydrate of 4-(2-chloro-4-methoxyphenyl)-5-methyl-2-[N-(2-ethoxynaphth-1-yl)-N-propylamino]thiazole;
chlorhydrate of 4-(2-chloro-4-methoxyphenyl)-5-methyl-2[N-(2,3-dimethylnaphth-1-yl)-N-propylamino]thiazole;
chlorhydrate de 4-(2-chloro-4-methoxyphenyl)-5-methyl-2-[N-(6-bromo-2-methoxynaphth-1-yl)-N-propylamino]thiazole;
chlorhydrate of 4-(2-chloro-4-methoxyphenyl)-5-methyl-2-[N-(2,6-dimethylnaphth-1-yl)-N-propylamino]thiazole;
chlorhydrate of 4-(2-chloro-4-methoxyphenyl)-5-methyl-2-[N-(1-(methoxymethyl)-1-(naphth-2-yl)methyl)-N-propylamino]thiazole;
chlorhydrate of 4-(2-chloro-4-methoxyphenyl)-5-methyl-2-[N-(1-(cyclopropyl)-1 -(naphth-2-yl)methyl)-N-propylamino]thiazole;
3-(2,4-dichlorophenyl)-5-methyl-7(N-propyl-N-cyclopropanemethylamino)-pyrazolo[2,3-a]pyrimidine;
3-(2,4-dichlorophenyl)-5-methyl-7-(N-allyl-N-cyclopropanemethylamino)-pyrazolo[2,3-a]pyrimidine;
2-methylthio-3-(2,4-dichlorophenyl)-5-methyl-7-(N,N-diallylamino) -pyrazolo[2,3-a]pyrimidine;
2-methylthio-3-(2,4-dichlorophenyl)-5-methyl-7-(N-butyl-N-cyclopropanemethyl-amino)pyrazolo[2,3-alpyrimidine;
2_methylthio-3-(2,4-dichlorophenyl)-5-methyl-7-(N-propyl-N-cyclopopanemethyl-amino) pyrazolo[2,3-a]pyrimidine;
2-methyl-3-(4-chlorophenyl)-5-methyl-7-(N,N-dipropylamino)-pyrazolo[2,3-a) pyrimidine;
3-[6-(dimethylamino)-3-pyridinyl-2,5-dimethyl-N,N-dipropylpyrazolo[2,3-a]pyrimidin-7-amine;
3-[6-(dimethylamino)-4-methyl-3-pyridinyl]-2,5-dimethyl-N,N-dipropyl-pyrazolo[2,3-a]p yrimidine-7-amine;
3-(2,4-dimethoxyphenyl)-2,5-dimethyl-7-(N-propyl-N-methyloxyethylamino)-pyrazolo (2,3-a)pyrimidine;
7-(N-diethylamino)-2,5-dimethyl-3-(2-methyl-4-methoxyphenyl-[1,5-a]-pyrazolopyrimidine;
7-(N-(3-cyanopropyl)-N-propylamino-2,5,dimethyl-3-(2,4-dimethylphenyl)-[1,5-a]-pyrazolopyrimidine;
[3,6-dimethyl-2-(2,4,6-trimethyl-phenoxy)-pyridin-4-yl]-(1-ethyl-propyl)-amine;
[2-(4-chloro-2,6-dimethyl-phenoxy)-3,6-dimethyl-pyridin-4-yl]-(1-ethyl-propyl)-amine; cyclopropylmethyl-[3-(2,4-dimethyl-phenyl)-2,5-dimethyl-pyrazolo[1,5-a]pyrimidin-7-yl]-propyl-amine;
cyclopropylmethyl-[3-(2-methyl-4-chloro-phenyl)-2,5-dimethyl-pyrazolo[1,5-a]pyrimidin-7-yl]-propyl-amine;
cyclopropylmethyl-[3-(2,4-di-chloro-phenyl)-2,5-dimethyl-pyrazolo[1,5-a]pyrimidin-7-yl]-propyl-amine;
[3-(2-methyl-4-chloro-phenyl)-2,5-dimethyl-pyrazolo[1,5-a]pyrimidin-7-yl]-di-propyl-amine;
[2,5-dimethyl-3-(2,4-dimethyl-phenyl)-pyrazolo[1,5-a]pyrimidin-7-yl]-(1-ethyl-propyl)-amine;
[2,5-dimethyl-3-(2,4-dichloro-phenyl)-pyrazolo[1,5-a]pyrimidin-7-yl]-(1-ethyl-propyl)-amine; and
4-(1-Ethyl-propylamino)-6-methyl-2-(2,4,6-trimethyl-phenoxy)-nicotinic acid methyl ester.

Methods for making the CRF antagonists described above are disclosed in the above-listed patents and published patent applications incorporated by reference herein.

In an alternative embodiment, the present invention relates to a pharmaceutical composition for the treatment of a condition selected from the group consisting of:
a) abnormal circadian rhythm; and
b) depression.
The composition comprises an amount of a CRF antagonist effective to treat the condition in combination with a pharmaceutically acceptable carrier. Where the condition is depression, it is also treated with a second compound for treating depression, the second compound having an onset of action that is delayed with respect to that of the CRF antagonist.

In another aspect, the present invention relates to a method for treating or preventing a cardiovascular disease that involves administering a CRF antagonist, or a pharmaceutically acceptable salt, isomer, or prodrug thereof, in combination with a second, non-CRF antagonist compound for treating the disease. The second compound for treating the disease can be, for example, adenosine, alteplase, amiodarone, anagrelide, ardeparin, argatroban, atenolol, atorvastatin, benazepril, captopril, carvedilol, cerivastatin, clonidine, clopidrogrel, dalteparin, danaparoid, diltiazem, enalapril, fluvastatin, fosinopril, gemfibrozil, hydrochlorothiazide, irbesartan, lepirudin, lisinopril, lovastatin, oprelvekin, pravastatin, prazosin, quinapril, ramipril, saruplase, simvastatin, terazosin, valsartan, or verapamil.

In another aspect, the invention relates to treatment of migraine or non-migraine headache by administration of a CRF antagonist in combination with a non-CRF antagonist compound that treats such condition. For example, it is possible to administer a CRF antagonist with non-steroidal anti-inflammatory drugs (NSAIDs), such as aspirin, acetaminophen, ibuprofen, with anti-emetics, with preparations containing ergotamine such as dihydroergotamine, or with agents that modulate serotonin receptors (including those that modulate the 5HT_{1B}, 5HT_{1D}, SHT_{1F} and 5HT_{2B} receptors) or that mimic the effects of serotonin. Particular agents include sumatriptan, naratriptan, zolmitriptan, rizatriptan, eletriptan, and almotriptan. Administration of these compounds is carried out using dosages and formulations that are well-known.

In another aspect, the invention relates to treatment of emesis using a CRF antagonist in combination with a non-CRF antagonist compound for treating emesis. Examples of such non-CRF antagonist compounds for treating emesis include tachykinin antagonists, including Nk1 antagonists, (such as compounds described in WO 99/24423, EP 867182, EP 980324, and WO 99/24423) and 5HT₃ antagonists (such as metoclopramide, granisetron, dolasetron, ondansetron and tropisetron).

The emesis that is treated can be of any type, including emesis induced by pregnancy, vestibular disorder, post-operative sickness, gastrointestinal obstruction, reduced gastrointestinal motility, visceral pain, migraine, change in intercranial pressure, chemotherapy, radiation, toxins, and opioid analgesics.

The invention also encompasses combined pharmaceutical compositions containing the CRF antagonist, a non-CRF antagonist as defined above, and below, and a pharmaceutically acceptable carrier. Examples of such compositions include, without limitation:
1) a composition for treating abnormal circadian rhythm that contains effective amounts of a combination of a CRF antagonist and a non-CRF antagonist compound useful for treating abnormal circadian rhythm;
2) a composition for treating depression that contains effective amounts of a combination of a CRF antagonist and a second compound for treating depression that has a delayed effect;
3) a composition for treating or preventing a cardiovascular disease that contains effective amounts of a CRF antagonist in combination with a second, non-CRF antagonist compound for treating the disease;
4) a composition for treating migraine or non-migraine headache that contains effective amounts of a CRF antagonist in combination with a non-CRF antagonist compound that treats such condition; and
5) a composition for treating emesis that contains a CRF antagonist in combination with a non-CRF antagonist compound for treating emesis.

Combination treatments according to the invention can be administered as part of the same pharmaceutical composition, or the active agents can be administered separately as part of an appropriate dose regimen designed to obtain the benefits of the combination therapy.

Acid addition salts of the CRF antagonists and other agents employed in the invention can be prepared in a conventional manner by treating a solution or suspension of the corresponding free base with one chemical equivalent of a pharmaceutically acceptable acid. Conventional concentration or crystallization techniques can be employed to isolate the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, p-toluenesulfonic, and related acids.

The CRF antagonists and their pharmaceutically acceptable salts, and any second pharmaceutically active compounds, may be administered alone or in combination with pharmaceutically acceptable carriers, in either single or multiple doses. Suitable pharmaceutical carriers include inert solid diluents or fillers, sterile aqueous solutions, oils (e.g., peanut oil, sesame oil) and various organic solvents. The pharmaceutical compositions formed by combining the CRF antagonists and pharmaceutically acceptable carriers can be readily administered in a variety of dosage forms such as tablets, powders, lozenges, emulsions, oil soft gels, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid, compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing the CRF antagonist or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosages for the CRF antagonists employed in the methods of this invention will depend on the intended route of administration and factors such as the age and weight of the patient, as generally known to a physician. The dosages will also depend on the particular condition to be treated and will generally range from about 0.1 to about 300 mg/kg body weight of the patient per day, with administration carried out in single or divided dosages.

Methods that may be used to determine the CRF antagonist activity of the compounds employed to practice the invention are described e.g., in Endocrinology, 116, 1653-1659 (1985) and Peptides, 10, 179-188 (1985).

Methods that can be used to determine the CRF binding protein inhibiting activity of compounds employed to practice the invention are described in Brain Research, (1997), 745(1,2), 248-256. The binding activities of the CRF antagonists employed generally range from about 0.5 nanomolar to about 32 micromolar.

## Claims

1. The compound [2-(4-chloro-2,6-dimethyl-phenoxy)-3,6-dimethyl-pyridin-4-yl]-(1-ethyl-propyl)-amine.
